(19) Europäisches Patentamt
European Patent Office
Office européen des brevets

(11) EP 3 950 119 A1

# (12) EUROPEAN PATENT APPLICATION
published in accordance with Art. 153(4) EPC

(43) Date of publication:
**09.02.2022 Bulletin 2022/06**

(21) Application number: **20785112.2**

(22) Date of filing: **27.03.2020**

(51) International Patent Classification (IPC):
***B01J 20/06*** (2006.01) ***B01J 20/08*** (2006.01)
***B01J 20/10*** (2006.01) ***B01J 20/28*** (2006.01)
***B01J 20/30*** (2006.01) ***A61M 1/02*** (2006.01)
***A61M 1/36*** (2006.01)

(52) Cooperative Patent Classification (CPC):
**A61M 1/02; A61M 1/36; B01J 20/06; B01J 20/08; B01J 20/10; B01J 20/28; B01J 20/30**

(86) International application number:
**PCT/JP2020/014383**

(87) International publication number:
**WO 2020/203926 (08.10.2020 Gazette 2020/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.03.2019 JP 2019068818**

(71) Applicant: **Asahi Kasei Medical Co., Ltd.**
**Tokyo 100-0006 (JP)**

(72) Inventors:
- **OISHI, Teruhiko**
**Tokyo 100-0006 (JP)**
- **TOKIMIZU, Yusuke**
**Tokyo 100-0006 (JP)**
- **MATSUYAMA, Keitaro**
**Tokyo 100-0006 (JP)**
- **MORITA, Naoki**
**Tokyo 100-0006 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

## (54) BLOOD PURIFIER AND METHOD FOR MANUFACTURING SAME

(57) Provided is a blood purifier which has a porous molded body with good phosphorus adsorption, which has good cytokine adsorption performance, no hemolysis, and can be used safely. The blood purifier has a porous molded body having a low-melting-point moisture content per gram of dry weight of 0.12 g or more and 1.35 g or less. After three and six months from sealing of a saline solution for injection into the blood purifier, the number of microparticles greater than or equal to 10 μm is 25 or less and the number of microparticles greater than or equal to 25 μm is three or less in 1 mL of the saline solution for injection.

FIG. 1

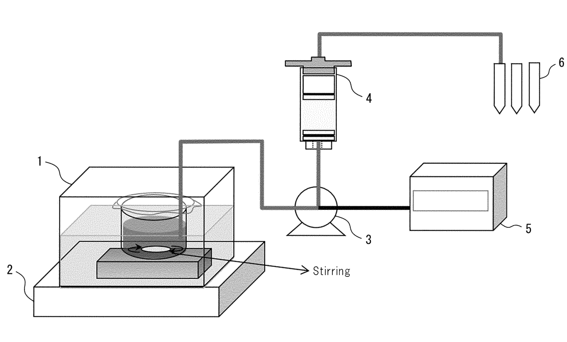


EP 3 950 119 A1

## Description

FIELD

The present invention relates to a blood purification device comprising a porous molded body and a method for producing it. More specifically, the invention relates to a blood purification device comprising a porous molded body having high phosphorus adsorption capacity and satisfactory cytokine adsorption performance, and no hemolysis, and that can be used safely, as well as to a method for producing it.

BACKGROUND

Healthy adults with normally functioning kidneys discharge excess phosphorous out of the body primarily through urine. However, kidney disease patients with impaired renal function, such as chronic renal failure patients, are unable to properly excrete excess phosphorus out of the body, and this leads to gradual internal buildup of phosphorus, causing the condition of hyperphosphatemia. Persistent hyperphosphatemia can lead to secondary hyperparathyroidism, resulting in renal osteopathy with symptoms such as painful and fragile or deformed bones that are prone to fracture, while in cases of concomitant hypercalcemia, the risk of cardiac failure due to calcification of the cardiovascular system also increases. Cardiovascular calcification is one of the most serious complications of chronic renal failure, and proper control of phosphorus levels in the body is extremely important to prevent hyperphosphatemia in chronic renal failure patients.

For hemodialysis patients, phosphorus that has accumulated in the body is periodically removed and regulated by dialysis treatment by hemodialysis, hemofiltration dialysis or hemofiltration so that hyperphosphatemia does not result. Dialysis treatment usually needs to be carried out three times per week, with a treatment period of 4 hours each time. However, when a hemodialysis patient ingests the 1000 mg of phosphorus that is usually ingested per day by a healthy person, the amount of phosphorus that would normally be excreted from the kidneys (650 mg) accumulates in the body, reaching an accumulated amount of 4550 mg within a week. Normal hemodialysis can remove about 800 to 1000 mg of phosphorus with a single dialysis procedure, allowing removal of about 3000 mg of phosphorus by dialysis 3 times a week. Since the amount of phosphorus that can be removed by dialysis treatment (3000 mg) does not match the amount of phosphorus that accumulates each week (4550 mg), accumulation of phosphorus in the body occurs as a result. Maintenance dialysis patients who are chronic renal failure patients have lost renal function as the major route of phosphorus excretion, and therefore the function of excreting phosphorus into the urine is essentially lost. Since phosphorus is not present in the dialysate from dialysis treatment it is possible to remove phosphorus from the body by diffusion into the dialysate, but at the current time it is not possible to achieve adequate excretion with the currently employed dialysis times and dialysis conditions. The phosphorus-removal effect of dialysis treatment alone is therefore inadequate, and consequently alimentary therapies and drug therapies with ingestion of phosphorus adsorbents are also used in addition to dialysis treatment to achieve phosphorus control, although it is important that consumption of phosphorus is restricted after having evaluated the nutritional status of the patient and confirmed that there is no malnutrition.

The CKD-MBD (chronic kidney disease-bone mineral metabolism disorder) guidelines for phosphorus control stipulate a serum phosphorus value of 3.5 to 6.0 mg/dL. A serum phosphorus level of below 3.5 mg/dL is hypophosphatemia which is a cause of rachitis or osteomalacia, while a level of 6.0 mg/dL or higher is hyperphosphatemia, which can lead to cardiovascular calcification. Alimentary therapy to lower phosphorus consumption also depends on the nutritional status of the patient, while the preferences of the patient must also be taken into account, and therefore management of body phosphorus concentrations with alimentary therapy can be difficult. Some drug therapies exist that are oral phosphorus adsorbents which can bind with dietary phosphate ion in the gastrointestinal tract to form insoluble phosphates, and which are taken either before or during meals to inhibit absorption of phosphorus through the intestinal tract, thus managing phosphorus concentrations. However, a very large amount of phosphorus adsorbent must be taken before meals for such drug therapy. This results in a high probability of side-effects when a phosphorus adsorbent is taken, such as vomiting, feeling of fullness, constipation or drug buildup in the body, such that the compliance is extremely low (often said to be 50% or lower), and therefore management of phosphorus concentrations by drugs can be problematic for both doctors and patients.

PTL 1 discloses circulating a dialysis composition containing a phosphorus adsorbent in dialysate during hemodialysis treatment to efficiently remove phosphorus in blood without direct contact of the phosphorus adsorbent with the blood.

Also, PTL 2 discloses a hemodialysis system wherein a phosphorus adsorbent comprising a polycationic polymer is provided separately from the hemodialyzer, whereby phosphorus accumulated in the blood is removed through the route of blood outside the body.

PTL 3 discloses a porous molded body suited as an adsorbent that can rapidly remove phosphorus and other

components by adsorption.

However, to date there has not been provided a blood purification device with a porous molded body that has high phosphorus adsorption capacity, no hemolysis, and safe usability.

[CITATION LIST]

[PATENT LITERATURE]

[PTL 1] International Patent Publication No. 2011/125758
[PTL 2] Japanese Unexamined Patent Publication No. 2002-102335
[PTL 3] Japanese Patent Publication No. 4671419

[NON PATENT LITERATURE]

[NPL 1] Shigeaki Morita, Masaru Tanaka and Yukihiro Ozaki, "Time-Resolved In Situ ATR-IR Observations of the Process of Sorption of Water into a Poly(2-methoxyethyl acrylate) Film", Langmuir, 2007, 23(7), Publication Date (web) March 3, 2007, pp.3750-3761
[NPL 2] T. Tsuruta, J. Biomater. et al., "The roles of water molecules in the interfaces between biological systems and polymers", Sci. Polym. Ed., 21, 2010, pp.1827-1920

SUMMARY

[TECHNICAL PROBLEM]

In light of these problems of the prior art, it is an object of the present invention to provide a blood purification device comprising a porous molded body, which has high phosphorus adsorption capacity, satisfy cytokine adsorption performance, no hemolysis, and safe usability.

[SOLUTION TO PROBLEM]

As a result of repeated experimentation with the aim of solving the problems described above, the present inventors have completed this invention upon finding that it is possible to provide a blood purification device having a high phosphorus clearance value, satisfactory cytokine adsorption performance and safe usability, by adding an inorganic ion adsorbent with high phosphorus adsorption capacity to a porous molded body, while washing with a supercritical fluid or subcritical fluid to completely remove the microparticles generated by the blood purification device comprising the porous molded body, without hemolysis.

Examples of embodiments of the invention are the following.

[1] A blood purification device comprising a porous molded body having a low-melting-point moisture content of 0.12 g to 1.35 g per gram of dry weight, wherein the number of microparticles of 10 μm or greater is 25 or less, and the number of microparticles of 25 μm or greater is 3 or less, in 1 mL of physiological saline for injection at 3 months and 6 months after sealing the physiological saline for injection in the blood purification device.
[2] The blood purification device according to [1] above, wherein the contact change rate of the porous molded body is from 0 to 0.2.
[3] The blood purification device according to [1] above, wherein the porous molded body is composed of a porous molded body-forming polymer, a hydrophilic polymer and an inorganic ion adsorbent.
[4] The blood purification device according to [3] above, wherein the hydrophilic polymer is a biocompatible polymer.
[5] The blood purification device according to [4] above, wherein the biocompatible polymer is a polyvinylpyrrolidone (PVP)-based polymer.
[6] The blood purification device according to any one of [1] to [5] above, wherein the porous molded body is coated with a biocompatible polymer.
[7] The blood purification device according to any one of [3] to [6] above, wherein the inorganic ion adsorbent contains at least one metal oxide represented by the following formula (1):

$$MN_xO_n \cdot mH_2O \quad (1)$$

where x is 0 to 3, n is 1 to 4, m is 0 to 6, and M and N are metal elements selected from the group consisting of Ti, Zr, Sn, Sc, Y, La, Ce, Pr, Nd, Sm, Eu, Gd, Tb, Dy, Ho, Er, Tm, Yb, Lu, Al, Si, Cr, Co, Ga, Fe, Mn, Ni, V, Ge, Nb and Ta, and are different from each other.

[8] The blood purification device according to [7] above, wherein the metal oxide is selected from among the following groups (a) to (c):

(a) hydrated titanium oxide, hydrated zirconium oxide, hydrated tin oxide, hydrated cerium oxide, hydrated lanthanum oxide and hydrated yttrium oxide;
(b) complex metal oxides comprising at least one metal element selected from the group consisting of titanium, zirconium, tin, cerium, lanthanum and yttrium and at least one metal element selected from the group consisting of aluminum, silicon and iron; and
(c) activated alumina.

[9] The blood purification device according to [1] above, having a phosphorus adsorption of 1.5 (mg-P/mL-resin) or greater.

[10] The blood purification device according to [1] above, having an adsorption rate for cytokines IL-1b, IL-6, IL-8 and IL-10 of 50% or greater.

[11] The blood purification device according to [1] above, having an adsorption rate for cytokine TNF-$\alpha$ is 30% or greater.

[12] The blood purification device according to [1] above, having an adsorption rate for alarmin HMGB-1 of 50% or greater.

[ADVANTAGEOUS EFFECTS OF INVENTION]

The blood purification device of the invention has high phosphorus adsorption capacity, satisfactory cytokine adsorption performance and no hemolysis, and can be used safely.

Specifically, the blood purification device of the invention has excellent selectivity and adsorption for phosphorus in blood even with a high blood flow rate during extracorporeal circulation treatment, and can eliminate the necessary amount of phosphorus from blood without affecting other components in the blood. Moreover, because phosphorus in blood can be effectively removed by extracorporeal circulation, phosphorous levels in blood can be properly managed without taking oral phosphorus adsorbents that produce side-effects.

By using the blood purification device of the invention, phosphorus levels in the blood of a dialysis patient can be properly managed without taking oral phosphorus adsorbents, or by taking only small amounts (auxiliary usage), thus avoiding side-effects in dialysis patients.

BRIEF DESCRIPTION OF DRAWINGS

Fig. 1 is an overview diagram of a column flow test apparatus for phosphorus adsorption by a blood purification device of the embodiment.
Fig. 2 shows an example of DSC measurement results for a porous molded body.
Fig. 3 is a diagram showing PMEA solubility with PMEA coating solution solvents.
Fig. 4 shows an example of ATR/FT-IR analysis of a porous molded body that includes PES and MOX, after PMEA coating.
Fig. 5 is a graph representing differences in PMEA coating amounts with PMEA coating solution solvents.

DESCRIPTION OF EMBODIMENTS

Embodiments of the invention will now be described in detail.

The blood purification device of the embodiment is a blood purification device comprising a porous molded body having a low-melting-point moisture content of 0.12 g to 1.35 g per gram of dry weight, wherein the number of microparticles of 10 $\mu$m or greater is 25 or less, and the number of microparticles of 25 $\mu$m or greater is 3 or less, in 1 mL of physiological saline for injection at 3 months and 6 months after sealing the physiological saline for injection in the blood purification device

[Low-melting-point moisture content]

For production of the blood purification device of the embodiment, it is necessary to adjust the low-melting-point moisture content per gram of dry weight of the porous molded body to the range of 0.12 g to 1.35 g, in order to obtain a blood purification device with high phosphorus adsorption capacity and no hemolysis. A porous molded body outside of this range will either have hemolysis, or its performance for phosphorus adsorption from the blood will be lower than desired.

It is known that a greater amount of intermediate water present on the surface of the polymer composing the porous molded body results in more excellent blood compatibility (see NPL 1, for example). Water adsorbed onto the surface of the polymer composing the porous molded body is classified as "non-freezing water" which strongly interacts with the polymer, "free water" which does not interact, and "intermediate water" which interacts weakly. Intermediate water differs entirely from ordinary 0°C-freezing water in its effect on biological interfaces, and the presence of intermediate water is thought to be important for materials with excellent blood compatibility. Generally speaking, "intermediate water" is defined as water that freezes at lower than 0°C (see NPL 2, for example). As a result of detailed analysis of water in porous molded bodies, however, the present inventors have found that the water that powerfully affects blood compatibility is water that freezes at lower than 0.18°C. For the purpose of the present specification, water that freezes at lower than 0.18°C is defined as "low-melting-point water".

Since it has conventionally been thought that the proportion of "intermediate water" on polymer surfaces is the water that affects biological interfaces, analysis of the water on the surface of the polymer composing the porous molded body has been focused only on the proportion of "intermediate water" with respect to ordinary water that freezes at 0°C. The present inventors have been the first to discover that the amount of "low-melting-point water", as water that freezes at lower than 0.18°C, is not only blood-compatible but also prevents hemolysis and affects cytokine adsorption rate and phosphorus adsorption.

[Estimated pore volume]

The cumulative pore volume for pores with pore diameters of 100 nm to 200 nm in the porous molded body is preferably 0.25 $cm^3/g$ or lower, more preferably 0.22 $cm^3/g$ or lower and even more preferably 0.20 $cm^3/g$ or lower. The cumulative pore volume is preferably within this range since it will allow the porous molded body to have more pores of sizes suited for adsorption of hydrophobic protein molecules, resulting in a porous molded body with more excellent adsorption properties. The cumulative pore volume is obtained by measuring the freeze-dried porous molded body by the nitrogen gas adsorption method and calculating by the BJH method.

[Albumin adsorption]

The albumin adsorption of the porous molded body is preferably 13 mg/mL to 90 mg/mL, more preferably 30 mg/mL to 90 mg/mL and even more preferably 45 mg/mL to 64 mg/mL. If the albumin adsorption is 13 mg/mL or greater then the cytokine adsorption performance of the porous molded body will be increased, and if the albumin adsorption is 90 mg/mL or lower then it will be possible to avoid reduction in albumin which is useful for the human body.

[Cytokine adsorption rate]

The adsorption rate of the porous molded body for cytokines other than TNF-$\alpha$ is 50% or greater, preferably 60% or greater and more preferably 70% or greater, which makes it possible to obtain a low-melting-point moisture content of 0.12 g or greater per gram of dry weight for the porous molded body. The adsorption rate for TNF-$\alpha$ is 30% or greater and preferably 60% or greater, which makes it possible to obtain a low-melting-point moisture content of 0.12 g or greater per gram of dry weight for the porous molded body.

A porous molded body comprising an inorganic ion adsorbent can significantly improve not only the adsorption rates for the cytokines mentioned above but also the adsorption rate for the alarmin High-Mobility Group Box 1 (HMGB1). According to one embodiment, a porous molded body including a specific inorganic ion adsorbent and a specific hydrophilic polymer, preferably a polyvinylpyrrolidone (PVP)-based polymer, can reduce the estimated volume of pores with pore diameters of 5 nm to 100 nm, and as a result can make it less likely to adsorb albumin that is useful for the human body. A porous molded body including a specific inorganic ion adsorbent and a specific hydrophilic polymer, preferably a polyvinylpyrrolidone (PVP)-based polymer, can also exhibit an HMGB1 adsorption rate of 90% or greater.

The adsorption rate for the alarmin High-Mobility Group Box 1 (HMGB1) is 60% or greater, preferably 65% or greater and more preferably 90% or greater, which makes it possible to achieve a low-melting-point moisture content of 0.12 g to 2.00 g per gram of dry weight for the porous molded body. It is not preferred for the low-melting-point moisture content per gram of dry weight of the porous molded body to exceed 2.00 g, because the HMGB1 adsorption rate will

tend to be lower than 60%. It is preferred to include a specific inorganic ion adsorbent in the porous molded body to allow the adsorption rate for the alarmin high-mobility group box 1 (HMGB1) to be 90% or greater.

[Contact change rate]

The contact change rate of the porous molded body of the embodiment is preferably 0% to 0.2%, more preferably 0% to 0.1% and even more preferably 0%. The contact change rate is the rate of change in mass when the porous molded body has been stirred and contacted with itself, causing it to partially fracture into microparticles and have reduced mass, and it is an index of the strength or fragility of the porous molded body. An accurate indicator of the strength or fragility of porous molded bodies has not existed in the prior art, but the present inventors found that if the contact change rate is higher than 0.2%, abrasion of the adsorbent increases during transport and use and the generated microparticles adversely affect organisms. If the contact change rate is limited to the range of 0% to 0.2%, then generation of microparticles can be inhibited and a blood purification device with superior safety can be provided.

In order to adjust the contact change rate to the range of 0% to 0.2%, in the case of a porous molded body obtained by coating a porous molded body with a hydrophilic polymer, the porous molded body prior to coating with the hydrophilic polymer is preferably composed of a hydrophobic polymer. In the case of an inorganic ion adsorbent-containing porous molded body, the molding slurry solution of the porous molded body preferably contains a hydrophilic polymer that is water-insoluble (meaning poorly soluble in water, which includes high-molecular-weight crosslinked structures). The molding slurry solution may also contain polyvinylpyrrolidone. Polyvinylpyrrolidone is water-soluble, but since it has high affinity with hydrophobic polymers, polyvinylpyrrolidone tends to remain in large amounts in the obtained porous molded body. Examples of polyvinylpyrrolidone include Polyvinylpyrrolidone K90 (product of BASF Corp. , weight-average molecular weight: 1,200,000). The porous molded body is more preferably coated with a hydrophilic polymer.

[Pore volume]

Since the pore volume of the porous molded body measured by the nitrogen gas adsorption method is a value primarily reflecting the pore volume of the inorganic ion adsorbent in the porous molded body, a larger value represents higher diffusion efficiency of ions into the inorganic ion adsorbent, and higher adsorption capacity. If the sum of the pore volumes per unit mass of the inorganic ion adsorbent is smaller than 0.05 $cm^3/g$, the pore volume of the inorganic ion adsorbent will be reduced and the adsorption capacity will be significantly lower. If the value is higher than 0.7 $cm^3/g$, on the other hand, the bulk density of the inorganic ion adsorbent will increase and the viscosity of the stock solution slurry will increase, thereby hampering granulation.

[Area-to-weight ratio]

For the embodiment, the area-to-weight ratio of the porous molded body measured by the nitrogen gas adsorption method is preferably 50 $m^2/g$ to 400 $m^2/g$, more preferably 70 $m^2/g$ to 350 $m^2/g$ and even more preferably 100 $m^2/g$ to 300 $m^2/g$. The area-to-weight ratio is obtained by measuring the freeze-dried porous molded body by the nitrogen gas adsorption method and calculating by the BET method. Since the area-to-weight ratio of the porous molded body measured by the nitrogen gas adsorption method is a value primarily reflecting the area-to-weight ratio of the inorganic ion adsorbent in the porous molded body, a larger value represents a greater number of ion adsorption sites and higher adsorption capacity. If the area-to-weight ratio of the porous molded body is smaller than 50 $m^2/g$, the number of adsorption sites of the inorganic ion adsorbent will be lower and the adsorption capacity will be significantly reduced. If the value is higher than 400 $m^2/g$, on the other hand, the bulk density of the inorganic ion adsorbent will increase and the viscosity of the stock solution slurry will increase, thereby hampering granulation.

[Loading mass of inorganic ion adsorbent]

For the embodiment, the loading mass of the inorganic ion adsorbent in the porous molded body is preferably 30 mass% to 95 mass%, more preferably 40 mass% to 90 mass% and even more preferably 50 mass% to 80 mass%. If the loading mass is less than 30 mass%, the contact frequency between the ions to be adsorbed and the inorganic ion adsorbent as the adsorption substrate will tend to be insufficient, while if it is greater than 95 mass%, the strength of the porous molded body will tend to be lacking.

[Mean particle size]

The porous molded body of the embodiment preferably has a mean particle size of 100 μm to 2500 μm and is essentially in the form of spherical particles, the mean particle size being preferably 150 μm to 2000 μm, more

preferably 200 μm to 1500 μm and even more preferably 300 μm to 1000 μm. The porous molded body of the embodiment is preferably in the form of spherical particles, although the spherical particles are not limited to being merely spherical and may also be elliptical spherical. The mean particle size for the embodiment is the median diameter of the sphere-equivalent size determined from the angular distribution of the intensity of scattered light due to laser light diffraction, assuming the porous molded body to be spherical. If the mean particle size is 100 μm or greater, pressure loss will be low when the porous molded body is packed into a container such as a column or tank, making it suitable for high-speed water treatment. If the mean particle size is 2500 μm or smaller, on the other hand, the surface area of the porous molded body can be increased when it has been packed into a column or tank, allowing reliable adsorption of ions even with high-speed liquid flow treatment.

[Inorganic ion adsorbent]

The porous molded body of this embodiment may also include an inorganic ion adsorbent. As used herein, "inorganic ion adsorbent" means an inorganic substance that exhibits an ion adsorption phenomenon or ion-exchange phenomenon. Examples of natural inorganic ion adsorbents include mineral substances such as zeolite and montmorillonite. Specific examples of mineral substances include kaolin minerals having a single layer lattice with aluminosilicates, muscovite, glauconite, kanuma soil, pyrophyllite and talc having a 2-layer lattice structure, and feldspar, zeolite and montmorillonite having a three-dimensional frame structure. Examples of synthetic-based inorganic ion adsorbents include metal oxides, polyvalent metal salts and insoluble hydrous oxides. Metal oxides include complex metal oxides, composite metal hydroxides and metal hydrous oxides.

From the viewpoint of adsorption performance for the target of absorption, and phosphorus, the inorganic ion adsorbent preferably contains at least one metal oxide represented by the following formula (1):

$$MN_xOn \cdot mH_2O \qquad (1)$$

{where x is 0 to 3, n is 1 to 4, m is 0 to 6, and M and N are metal elements selected from the group consisting of Ti, Zr, Sn, Sc, Y, La, Ce, Pr, Nd, Sm, Eu, Gd, Tb, Dy, Ho, Er, Tm, Yb, Lu, Al, Si, Cr, Co, Ga, Fe, Mn, Ni, V, Ge, Nb and Ta, and are different from each other}.

The metal oxide may be a non-water-containing (non-hydrated) metal oxide where m in formula (1) is 0, or it may be a hydrous metal oxide (hydrated metal oxide) wherein m is a numerical value other than 0. A metal oxide where x in formula (1) is a numerical value other than 0 is a complex metal oxide represented by the chemical formula in which each metal element is evenly distributed in a regular manner throughout all of the oxides, and the compositional ratio of the metal elements in the metal oxide is constant. Specific ones include nickel ferrite ($NiFe_2O_4$) or hydrous ferrite of zirconium ($Zr \cdot Fe_2O_4 \cdot mH_2O$, where m is 0.5 to 6), which form a perovskite structure or spinel structure. The inorganic ion adsorbent may also contain more than one type of metal oxide represented by formula (1).

From the viewpoint of excellent adsorption performance for components to be adsorbed, and especially phosphorus, a metal oxide as the inorganic ion adsorbent is preferably selected from among the following groups (a) to (c):

(a) hydrated titanium oxide, hydrated zirconium oxide, hydrated tin oxide, hydrated cerium oxide, hydrated lanthanum oxide and hydrated yttrium oxide,
(b) complex metal oxides comprising at least one metal element selected from the group consisting of titanium, zirconium, tin, cerium, lanthanum and yttrium and at least one metal element selected from the group consisting of aluminum, silicon and iron, and
(c) activated alumina.

It may be a material selected from among any of groups (a) to (c), or materials selected from among any of groups (a) to (c) may be used in combination, or materials of each of groups (a) to (c) may be used in combination. When materials are used in combination, they may be a mixture of two or more materials selected from among any of groups (a) to (c), or they may be a mixture of two or more materials selected from among two or more of groups (a) to (c).

From the viewpoint of low cost and high adsorption properties, the inorganic ion adsorbent may contain aluminum sulfate-added activated alumina.

From the viewpoint of inorganic ion adsorption properties and production cost, the inorganic ion adsorbent is more preferably one having a metal element other than M and N in solid solution in addition to the metal oxide represented by formula (1). For example, it may be one with iron in solid solution with hydrated zirconium oxide represented by $ZrO_2 \cdot mH_2O$ (where m is a numerical value other than 0).

Examples of salts of polyvalent metals include hydrotalcite-based compounds represented by the following formula (2):

$$M^{2+}_{(1-p)}M^{3+}_{p}(OH^{-})_{(2+p-q)}(A^{n-})_{q/r} \quad (2)$$

{where $M^{2+}$ is at least one divalent metal ion selected from the group consisting of $Mg^{2+}$, $Ni^{2+}$, $Zn^{2+}$, $Fe^{2+}$, $Ca^{2+}$ and $Cu^{2+}$, $M^{3+}$ is at least one trivalent metal ion selected from the group consisting of $Al^{3+}$ and $Fe^{3+}$, $A^{n-}$ is an n-valent anion, $0.1 \leq p \leq 0.5$, $0.1 \leq q \leq 0.5$, and r is 1 or 2}. A hydrotalcite-based compound represented by formula (2) is preferred because it is inexpensive as an inorganic ion adsorbent and has high adsorption properties.

Examples of insoluble hydrous oxides include insoluble heteropolyacid salts and insoluble hexacyanoferrates. A metal carbonate as the inorganic ion adsorbent has excellent performance from the viewpoint of adsorption, but using a carbonate requires consideration from the viewpoint of elution. From the viewpoint of allowing ion-exchange reaction with the carbonate ion, the metal carbonate may include at least one type of metal carbonate represented by the following formula (3):

$$QyRz(CO_3)s \cdot tH_2O \quad (3)$$

{where y is 1 or 2, Z is 0 or 1, s is 1 to 3, t is 0 to 8, and Q and R are metal elements selected from the group consisting of Mg, Ca, Sr, Ba, Sc, Mn, Fe, Co, Ni, Ag, Zn, Y, La, Ce, Pr, Nd, Sm, Eu, Gd, Tb, Dy, Ho, Er, Tm, Yb and Lu and are different from each other}. The metal carbonate may be a non-hydrous (non-hydrated) metal carbonate where t in formula (3) is 0, or it may be a hydrate where t is an integer other than 0.

From the viewpoint of low elution and excellent adsorption properties for phosphorus, boron, fluorine and/or arsenic, the inorganic ion adsorbent is preferably selected from among the following group (d):

(d) magnesium carbonate, calcium carbonate, strontium carbonate, barium carbonate, scandium carbonate, manganese carbonate, iron carbonate, cobalt carbonate, nickel carbonate, silver carbonate, zinc carbonate, yttrium carbonate, lanthanum carbonate, cerium carbonate, praseodymium carbonate, neodymium carbonate, samarium carbonate, europium carbonate, gadolinium carbonate, terbium carbonate, dysprosium carbonate, holmium carbonate, erbium carbonate, thulium carbonate, ytterbium carbonate and lutetium carbonate.

The inorganic ion adsorption mechanism for the metal carbonate is expected to include elution of the metal carbonate and recrystallization of inorganic ions and metal ions on the metal carbonate, and therefore a higher degree of solubility of the metal carbonate is anticipated to result in higher inorganic ion adsorption and more excellent adsorption performance. Metal elution from the inorganic ion adsorbent is also a concern, and therefore careful study is necessary for uses where metal elution may be a problem.

The inorganic ion adsorbent composing the porous molded body of the embodiment may also contain contaminating impurity elements that are present due to the production process, in ranges that do not interfere with functioning of the porous molded body. Examples of potentially contaminating impurity elements include nitrogen (in the form of nitric acid, nitrous acid or ammonium), sodium, magnesium, sulfur, chlorine, potassium, calcium, copper, zinc, bromine, barium and hafnium.

The method of replacement to organic liquid is not particularly restricted, and it may be centrifugal separation and filtration after dispersing the water-containing inorganic ion adsorbent in an organic liquid, or passage of an organic liquid after filtration with a filter press. For a higher replacement rate, it is preferred to repeat a method of filtration after dispersion of the inorganic ion adsorbent in an organic liquid.

The replacement rate of water to organic liquid during production may be 50 mass% to 100 mass%, preferably 70 mass% to 100 mass% and more preferably 80 mass% to 100 mass%. The organic liquid replacement rate is the value represented by the following formula (4):

$$Sb = 100 - Wc \quad (4)$$

where Sb (mass%) is the replacement rate to organic liquid and Wc (mass%) is the moisture content of the filtrate after treating the water-containing inorganic ion adsorbent with the organic liquid.

The moisture content of the filtrate after treatment with the organic liquid can be determined by measurement by the Karl Fischer method.

Drying after replacement of the water in the inorganic ion adsorbent with organic liquid can inhibit aggregation during drying, can increase the pore volume of the inorganic ion adsorbent and can increase the adsorption capacity. If the replacement rate of the organic liquid is less than 50 mass%, the aggregation suppressing effect during drying will be reduced and the pore volume of the inorganic ion adsorbent will not increase.

The sum Va of the pore volumes per unit mass of the inorganic ion adsorbent is determined by the following formula (7):

$$Va = Vb/Sa \times 100 \quad (7)$$

where Vb ($cm^3/g$) is the pore volume per unit mass of the porous molded body calculated for the dried porous molded body and Sa (mass%) is the loading mass of the inorganic ion adsorbent in the porous molded body.

The loading mass (mass%) Sa of the inorganic ion adsorbent in the porous molded body is determined by the following formula (8):

$$Sa = Wb/Wa \times 100 \ (8)$$

where Wa (g) is the mass of the porous molded body when dry and Wb (g) is the ash content mass.

The ash content is the portion remaining after the porous molded body has been fired at 800°C for 2 hours.

[Removal of microparticles]

The blood purification device of the embodiment can be safely used even though the porous molded body contains an inorganic ion adsorbent. Specifically, in the blood purification device of the embodiment, the number of microparticles of 10 μm or greater is no more than 25 and the number of microparticles of 25 μm or greater is no more than 3, in 1 mL of the physiological saline for injection at 3 months and 6 months after the physiological saline for injection has been encapsulated in the blood purification device, while the absorbance of an eluate test solution is 0.1 or lower, and the test solution does not contain a membrane pore retainer.

The present inventors have found that even though the porous molded body contains an inorganic ion adsorbent when the blood purification device of the embodiment is produced, microparticles generated by the blood purification device can be completely removed if it is washed with a supercritical fluid or subcritical fluid.

A supercritical fluid is a fluid in a state above the critical pressure (hereunder also referred to as "Pc") and above the critical temperature (hereunder also referred to as "Tc"). A subcritical fluid is a fluid in a state other than a supercritical state, with conditions of $0.5 < P/Pc < 1.0$ and $0.5 < T/Tc$, or $0.5 < P/Pc$ and $0.5 < T/Tc < 1.0$, where the pressure and temperature during reaction are denoted by P and T, respectively. The preferred ranges for the pressure and temperature of the subcritical fluid are $0.6 < P/Pc < 1.0$ and $0.6 < T/Tc$, or $0.6 < P/Pc$ and $0.6 < T/Tc < 1.0$. When the fluid is water, the ranges for the temperature and pressure for a subcritical fluid may be $0.5 < P/Pc < 1.0$ and $0.5 < T/Tc$, or $0.5 < P/Pc$ and $0.5 < T/Tc < 1.0$. The temperature is represented as degrees Celsius, and the formula representing the subcritical state does not apply if either Tc or T is a negative value.

The supercritical fluid or subcritical fluid used may be water or an organic medium such as alcohol, or a gas such as carbon dioxide, nitrogen, oxygen, helium, argon or air, or a mixed fluid comprising them. Carbon dioxide is most preferred because it allows a supercritical state to be achieved at nearly ordinary temperature, so that various different substances can be thoroughly dissolved.

[Porous molded body-forming polymer]

Porous molded body-forming polymers that can form porous molded bodies to be used in the blood purification device of this embodiment are not particularly limited, and examples include various types such as polysulfone-based polymers, polyvinylidene fluoride-based polymers, polyvinylidene chloride-based polymers, acrylonitrile-based polymers, polymethyl methacrylate-based polymers, polyamide-based polymers, polyimide-based polymers, cellulosic polymers, ethylene-vinyl alcohol copolymer-based polymers, polyaryl ether sulfones, polypropylene-based polymers, polystyrene-based polymers and polycarbonate-based polymers. Among these, aromatic polysulfones are preferred for excellent thermostability, acid resistance, alkali resistance and mechanical strength.

Aromatic polysulfones to be used for the embodiment include those having repeating units represented by the following formula (5):

$$-O-Ar-C(CH_3)_2-Ar-O-Ar-SO_2-Ar- \quad (5)$$

{where Ar is a phenyl group disubstituted at the para positions}
or the following formula (6):

$$-O-Ar-SO_2-Ar- \quad (6)$$

{where Ar is a phenyl group disubstituted at the para positions}. The polymerization degree and molecular weight of the

aromatic polysulfone are not particularly restricted.

[Hydrophilic polymer]

The hydrophilic polymer in the porous molded body is not particularly restricted so long as it is a biocompatible polymer that swells in water but does not dissolve in water. Throughout the present specification, "hydrophilic polymer" will also be referred to as "biocompatible polymer". Hydrophilic polymers include polymers having one or more from among sulfonate, carboxyl, carbonyl, ester, amino, amide, cyano, hydroxyl, methoxy, phosphate, oxyethylene, imino, imide, imino ether, pyridine, pyrrolidone, imidazole and quaternary ammonium groups.

When the hydrophobic polymer is an aromatic polysulfone, a polyvinylpyrrolidone (PVP)-based polymer is most preferred as the hydrophilic polymer. Polyvinylpyrrolidone-based polymers include vinylpyrrolidone-vinyl acetate copolymer, vinylpyrrolidone-vinylcaprolactam copolymer and vinylpyrrolidone-vinyl alcohol copolymer, and preferably at least one of these is used as the hydrophilic polymer. From the viewpoint of compatibility with polysulfone-based polymers and polyether sulfone-based polymers, the most suitable ones for use are polyvinylpyrrolidone, vinylpyrrolidone-vinyl acetate copolymer and vinylpyrrolidone-vinylcaprolactam copolymer.

The hydrophilic polymer is preferably a polymer that includes a monomer represented by chemical formula (1) as a monomer unit.

[Chemical Formula 1]

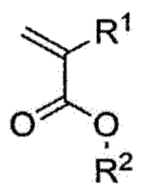

(1)

In chemical formula (1), $R^1$ is a hydrogen atom or methyl group, $R^2$ is $-CH_2(CH_2)_qOC_tH_{2t+1}$ or $-CH_2C_mH_{2m+1}$, q is 1 to 5, t is 0 to 2 and m is 0 to 17.

The monomer represented by chemical formula (1) is preferably one or more selected from the group consisting of 2-hydroxyethyl methacrylate (HEMA), 2-methoxyethyl methacrylate (MEMA), *n*-butyl methacrylate (BMA) and lauryl methacrylate (LMA), and more preferably 2-methoxyethyl methacrylate (MEMA). These monomers are preferred because they will allow higher excess adsorption to be maintained on the porous molded body, and can improve blood compatibility.

According to one embodiment, the porous molded body preferably has the hydrophilic polymer (biocompatible polymer) supported on the hydrophobic polymer porous molded body, and more preferably a biocompatible polymer represented by chemical formula (1) is supported.

The content of the monomer represented by chemical formula (1) is preferably 40 mol% or greater and more preferably 60 mol% or greater, based on the entire amount of monomers composing the biocompatible polymer. The upper limit for the monomer content is not limited, and it may be 100 mol%, or 80 mol% or less or 60 mol% or less, based on the entire amount of monomers composing the biocompatible polymer.

The biocompatible polymer preferably further includes as another monomer unit, a charged monomer that is copolymerizable with the monomer represented by chemical formula (1). As used herein, "charged monomer" is a monomer having a functional group that is partially or completely charged with a positive charge or negative charge under conditions of pH 7.0. If the biocompatible polymer further includes a charged monomer as a monomer unit, then its use in combination with the porous molded body will lower the loading mass of biocompatible polymer supported on the porous molded body and can help prevent reduction in adsorption. The charged monomer also has increased biocompatibility due to its high hydrophilicity. This tends to result in a porous molded body that has more satisfactory adsorption and blood compatibility.

The charged monomer may be, for example, a monomer with at least one group selected from the group consisting of amino groups ($-NH_2$, $-NHR^3$, $NR^3R^4$), carboxyl groups (-COOH), phosphate groups ($-OPO_3H_2$), sulfonate groups ($-SO_3H$) and zwitterionic groups. For amino groups, preferably $R^3$ and Rare each independently an alkyl group of 1 to 3 carbon atoms, and more preferably an alkyl group of 1 or 2 carbon atoms.

Among these, the charged monomer is more preferably a monomer having at least one group selected from the group consisting of amino, carboxyl and zwitterionic groups. The charged monomer even more preferably is at least one selected from the group consisting of cationic monomers with amino groups, anionic monomers with carboxyl groups,

zwitterionic monomers with amino groups and carboxyl groups, and zwitterionic monomers with amino groups and phosphate groups. The charged monomer even more preferably has a carboxyl group from the viewpoint of adsorption of $Ca^{2+}$ by the porous molded body, and inhibiting acceleration of blood clotting.

More specifically, the charged monomer is more preferably at least one selected from the group consisting of 2-aminoethyl methacrylate (AEMA), dimethylaminoethyl methacrylate (DMAEMA), diethylaminoethyl methacrylate (DEAEMA), [2-(methacryloyloxy)ethyl]trimethylammonium, acrylic acid (AAc), methacrylic acid (MAc), 2-(methacryloyloxy)ethyl phosphate, N-methacryloyloxyethyl-N,N-dimethylammonium-α-N-methylcarboxybetaine (CMB), [2-(methacryloyloxy)ethyl]dimethyl-(3-sulfopropyl)ammonium hydroxide (SPB), [3-(methacryloylamino)propyl]dimethyl(3-sulfopropyl)ammonium hydroxide (SPBA), 2-(methacryloyloxy)ethyl 2-(trimethylammonio)ethyl phosphate (MPC) and [3-(methacryloylamino)propyl]dimethyl(3-sulfobutyl)ammonium.

Among these, the charged monomer is more preferably at least one selected from the group consisting of methylaminoethyl methacrylate (DMAEMA), diethylaminoethyl methacrylate (DEAEMA), acrylic acid (AAc), N-methacryloyloxyethyl-N,N-dimethylammonium-α-N-methylcarboxybetaine (CMB) and 2-(methacryloyloxy)ethyl 2-(trimethylammonio)ethyl phosphate (MPC), and even more preferably N-methacryloyloxyethyl-N,N-dimethylammonium-α-N-methylcarboxybetaine (CMB).

The content of the charged monomer is preferably 10 mol% to 60 mol% and more preferably 15 mol% to 40 mol%, based on the entire amount of monomers composing the biocompatible polymer. If the charged monomer content is within this range, the balance between the impregnating property and hydrophilicity of the porous molded body will tend to be excellent, and a porous molded body with superior adsorption and biocompatibility will tend to be obtained.

The weight-average molecular weight (Mw) of the biocompatible polymer is preferably 5,000 to 5,000,000, more preferably 10,000 to 1,000,000 and even more preferably 10,000 to 300,000. The weight-average molecular weight of the biocompatible polymer is preferably within this range from the viewpoint of suitable impregnation into the porous molded body, preventing elution into blood, and reducing the loading mass. The method of analyzing the weight-average molecular weight (Mw) of the biocompatible polymer may be measurement by gel permeation chromatography (GPC), for example.

The biocompatibility (blood compatibility) of PMEA is described in detail in "Artificial organ surface-biocompatibilizing materials", Tanaka, K., BIO INDUSTRY, Vol 20, No.12, 59-70 2003.

It is known that in the ATR-IR method, waves impinging on a sample are reflected after entering into the sample to a small degree, such that infrared absorption in the region of the entering depth can be measured, but the present inventors have found that the region of measurement in the ATR-IR method is essentially equal to the depth of the "surface layer" that corresponds to the surface of the porous molded body. That is, it was found that the blood compatibility in a region at approximately equal depth as the ATR-IR measurement region governs the blood compatibility of the porous molded body, and that the presence of PMEA in that region can provide a blood purification device with consistent blood compatibility. If the surface of the porous molded body is coated with PMEA, then generation of microparticles from the blood purification device after long-term storage can also be inhibited.

The measuring region by ATR-IR depends on the wavelength and incident angle of infrared light in air, the refractive index of the prism and the refractive index of the sample, but it will usually be a region of within 1 μm from the surface.

The presence of PMEA on the surface of the porous molded body can be confirmed by thermal decomposition gas chromatography-mass spectrometry of the porous molded body. The presence of PMEA is estimated using the peak near 1735 $cm^{-1}$ on the infrared absorption curve from total reflection infrared absorption (ATR-IR) measurement of the surface of the porous molded body, although neighboring peaks can arise due to other substances. Thermal decomposition gas chromatography-mass spectrometry may therefore be performed to confirm the presence of PMEA, by confirming PMEA-derived 2-methoxyethanol.

PMEA has a characteristic solubility in solvents. For example, PMEA does not dissolve in a 100% ethanol solvent or 100% methanol solvent but has a range of solubility in a water/ethanol mixed solvent or water/methanol mixed solvent, depending on the mixing ratio. If the mixing ratio is in the soluble range, the peak intensity of the PMEA-attributed peak (near 1735 $cm^{-1}$) is higher with a larger amount of water. From the viewpoint of the PMEA solubility and the PMEA coating amount, the methanol:water ratio is preferably 80:20 to 40:60, more preferably 70:30 to 45:55 and even more preferably 60:40 to 45:55.

For a porous molded body comprising PMEA on the surface, the variation in water permeability is minimal and product design is simpler, due to lower variation in pore sizes on the surface. The porous molded body of this embodiment has PMEA on the surface, but when the PMEA has been coated onto the porous molded body it is assumed that the PMEA adheres as an ultra-thin film, coating the porous molded body surface essentially without blocking the pores. PMEA is especially preferred because of its small molecular weight and short molecular chains, which makes it less likely to form a thick coating film structure or to alter the structure of the porous molded body. PMEA is also preferred because it has high compatibility with other substances, allowing it to be evenly coated onto the porous molded body surface and helping to improve the blood compatibility.

Hemolysis of the porous molded body can be eliminated by evenly coating PMEA onto the surface of the porous molded body using a water/methanol mixed solvent that contains PMEA.

The method of forming a PMEA coating layer on the surface of the porous molded body may be a method of coating by flowing a PMEA-dissolved coating solution from the top of a column (vessel) packed with the porous molded body.

The polyvinylpyrrolidone (PVP)-based polymer is not particularly restricted, but polyvinylpyrrolidone (PVP) is suitable for use.

[Number of microparticles]

A blood purification device that is to be applied for dialysis must conform to the approval standards for artificial kidney devices established by the Ministry of Health, Labour and Welfare, in order to obtain approval for production as a dialysis-type artificial kidney device. The blood purification device of the embodiment must therefore conform to the eluting material test criteria listed in the approval standards for artificial kidney devices. In the blood purification device of the embodiment, the number of microparticles of 10 μm or greater is no more than 25 in 1 mL of saline solution and the number of microparticles of 25 μm or greater is no more than 3 in 1 mL of saline solution, at 3 and 6 months after sealing of the physiological saline for injection in the blood purification device, while the absorbance of the eluate test solution is 0.1 or lower.

The method of measuring the number of microparticles in the physiological saline for injection encapsulated in the blood purification device is as follows.

(1) Measuring method for wet-type blood purification device

A wet-type blood purification device encapsulates a solution (such as UF filtration membrane water) just before shipping and is subjected to radiation sterilization in the solution, and then shipped. In a wet-type blood purification device, after the solution has been completely removed and after the porous molded body in the blood purification device has been flushed with 10 L of physiological saline for injection (or after filtering from the membrane inner surface side to the membrane outer surface side if the porous molded body is a hollow fiber membrane), fresh physiological saline for injection is encapsulated, and then the mixture is incubated at 25°C±1°C and stored in a stationary state for 3 months. Sampling of the saline solution from the blood purification device is carried out after removing as much of the solution (filled solution) as possible from the blood purification device and then uniformly mixing. For example, after sampling for measurement at 3 months, the remaining saline solution is placed in the original blood purification device and sealed, stored for an additional 3 months, and used for measurement at 6 months.

(2) Measuring method for dry-type blood purification device

With a dry-type blood purification device, radiation sterilization is usually not carried out in solution, and it is usually shipped in a dry state. After the porous molded body in the blood purification device has been flushed with 10 L of physiological saline for injection (or after filtering from the membrane inner surface side to the membrane outer surface side if the porous molded body is a hollow fiber membrane), fresh physiological saline for injection is encapsulated, and then the mixture is incubated at 25°C ±1°C and stored in a stationary state for 3 months. Sampling of the saline solution from the blood purification device is carried out after removing as much of the solution (filled solution) as possible from the blood purification device and then uniformly mixing. For example, after sampling for measurement at 3 months, the remaining saline solution is placed in the original blood purification device and sealed, stored for an additional 3 months, and used for measurement at 6 months. The number of microparticles in the sampled solution (or filled solution) can be measured with a particle counter.

[Phosphorus adsorption performance of porous molded body]

The porous molded body of the embodiment can be suitably used for adsorption of phosphorus during hemodialysis of a dialysis patient. The composition of blood is categorized into blood plasma components and blood cell components, with the blood plasma components comprising 91% water, 7% proteins, and lipid components and inorganic salts, and with phosphorus in the blood being present as phosphate ions among the blood plasma components. The blood cell components are composed of 96% erythrocytes, 3% leukocytes and 1% platelets, the sizes of erythrocytes being 7 to 8 μm in diameter, the sizes of leukocytes being 5 to 20 μm in diameter and the sizes of platelets being 2 to 3 μm in diameter.

Since the most common pore size of a porous molded body measured by a mercury porosimeter is 0.08 to 0.70 μm, and consequently the abundance of the inorganic ion adsorbent on the outer surface is high, this allows

phosphorus ions to be reliably adsorbed even by high-speed liquid flow treatment, and also allows excellent penetration, diffusion and adsorption of phosphorus ions into the porous molded body. There is also no reduction in blood flow by clogging with blood cell components. For this embodiment, the surface of the porous molded body has a biocompatible polymer, allowing it to be used as a more suitable phosphorus adsorbent for blood treatment.

If the device comprises a porous molded body with the most abundant pore size being 0.08 to 0.70 μm, and the surface of the porous molded body has a biocompatible polymer, then phosphorus ions in blood will be selectively and reliably adsorbed, so that the phosphorus concentration in blood returning to the body will be nearly 0. By returning essentially phosphorus-free blood to the body, presumably phosphorus will more actively move into the blood from intracellular or extracellular regions, for a greater refilling effect. By inducing a refilling effect to supplement phosphorus in the blood, it becomes possible to eliminate even phosphorus present in extracellular fluid or in cells, which normally cannot be eliminated. Thus, phosphorus levels in the blood of a dialysis patient can be properly managed without taking oral phosphorus adsorbents, or by taking only small amounts (auxiliary usage), thus avoiding side-effects in dialysis patients.

A blood purification device having a porous molded body packed into a vessel (column) may be used during dialysis in connection with a dialyzer, either in series before and after or in parallel with it. The blood purification device of the embodiment can be used as a blood purification device for adsorption of phosphorus, and has excellent selectivity and adsorption performance for inorganic phosphorus even with a low phosphorus level in the blood and a high space velocity. From the viewpoint of helping to induce a refilling effect, preferably the blood purification device of the embodiment is used in connection before and after the dialyzer.

From the viewpoint of allowing a refilling effect to be obtained, the phosphorus adsorption rate (%) (the proportion of blood phosphorus that is absorbed) is preferably 50% or higher, more preferably 60% or higher, and most suitably 70% or higher, 80% or higher, 85% or higher, 90% or higher, 95% or higher or 99% or higher.

There are no limitations on the material of the vessel (column) of the blood purification device of the embodiment, and examples are mixed resins such as polystyrene-based polymers, polysulfone-based polymers, polyethylene-based polymers, polypropylene-based polymers, polycarbonate-based polymers and styrene-butadiene blocked copolymers. A polyethylene-based polymer or polypropylene-based polymer is preferably used from the viewpoint of material cost.

[Method for producing porous molded body]

The method for producing a porous molded body of the embodiment will now be described in detail.

The method for producing a porous molded body of the embodiment includes, for example, (1) a step of drying an inorganic ion adsorbent, (2) a step of pulverizing the inorganic ion adsorbent obtained in step (1), (3) a step of mixing the inorganic ion adsorbent obtained in step (2), a good solvent for the porous molded body-forming polymer, a porous molded body-forming polymer and, depending on the case, a hydrophilic polymer (water-soluble polymer) to prepare a slurry, (4) a step of molding the slurry obtained in step (3), and (5) a step of coagulating the molded article obtained in step (4) in a poor solvent.

Step (1): Inorganic ion adsorbent drying step

In step (1), the inorganic ion adsorbent is dried to obtain a powder. In order to inhibit aggregation during the drying, preferably the drying during production is carried out after replacing the moisture with an organic liquid. The organic liquid is not particularly restricted so long as it has an effect of inhibiting aggregation of the inorganic ion adsorbent, but it is preferred to use a liquid with high hydrophilicity. Examples include alcohols, ketones, esters and ethers.

The replacement rate to organic liquid may be 50 mass% to 100 mass%, preferably 70 mass% to 100 mass% and more preferably 80 mass% to 100 mass%. The method of replacement to organic liquid is not particularly restricted, and it may be centrifugal separation and filtration after dispersing the water-containing inorganic ion adsorbent in an organic liquid, or passage of an organic liquid after filtration with a filter press. For a higher replacement rate, it is preferred to repeat a method of filtration after dispersion of the inorganic ion adsorbent in an organic liquid. The replacement rate to the organic liquid can be determined by measurement of the filtrate moisture content by the Karl Fischer method.

Drying after replacement of the water in the inorganic ion adsorbent with organic liquid can inhibit aggregation during drying, can increase the pore volume of the inorganic ion adsorbent and can increase the adsorption capacity. If the replacement rate of the organic liquid is less than 50 mass%, the aggregation suppressing effect during drying will be reduced and the pore volume of the inorganic ion adsorbent will not increase.

Step (2): Inorganic ion adsorbent pulverizing step

In step (2), the inorganic ion adsorbent powder obtained from step (1) is pulverized. The pulverizing method is not particularly restricted, and may be dry grinding or wet grinding. A dry grinding method is not particularly restricted,

and it may be one employing an impact crusher such as a hammer mill, an airflow pulverizer such as a jet mill, a medium pulverizer such as a ball mill or a compression pulverizer such as a roller mill. An airflow pulverizer is preferred among these because it can create a sharp particle size distribution for the pulverized inorganic ion adsorbent. A wet grinding method is not particularly restricted so long as it allows pulverizing and mixing together of the inorganic ion adsorbent and the good solvent for the porous molded body-forming polymer, and it may employ means used in physical pulverizing methods such as pressurized disruption, mechanical grinding or ultrasonic treatment.

Specific examples of pulverizing and mixing means include blenders such as generator shaft homogenizers and Waring blenders, medium agitation mills such as sand mills, ball mills, attritors and bead mills, and jet mills, mortar/pestle combinations, kneaders and sonicators. A medium agitation mill is preferred for high pulverizing efficiency and to allow pulverizing to a highly viscous state.

The ball diameter used in a medium agitation mill is not particularly restricted but is preferably 0.1 mm to 10 mm. If the ball diameter is 0.1 mm or greater, the ball mass will be sufficient to provide pulverizing force and high pulverizing efficiency, while a ball diameter of 10 mm or smaller will result in excellent fine pulverizing power.

The material of the ball used in a medium agitation mill is not particularly restricted, and it may be a metal such as iron or stainless steel, or a ceramic which is an oxide such as alumina or zirconia or a non-oxide such as silicon nitride or silicon carbide. Zirconia is superior among these for its excellent abrasion resistance, and from the viewpoint of low contamination (wear contamination) into products.

After pulverizing, a filter or the like is preferably used for filtration purification with the inorganic ion adsorbent in a fully dispersed state in the good solvent for the porous molded body-forming polymer. The particle size of the pulverized and purified inorganic ion adsorbent is 0.001 to 10 $\mu$m, preferably 0.001 to 2 $\mu$m and more preferably 0.01 to 0.1 $\mu$m. A smaller particle size is more favorable for uniformly dispersing the inorganic ion adsorbent in the membrane-forming solution. It tends to be difficult to produce uniform microparticles with sizes of smaller than 0.001 $\mu$m. With an inorganic ion adsorbent exceeding 10 $\mu$m, it tends to be difficult to stably produce a porous molded body.

Step (3): Slurry preparation step

In step (3), the inorganic ion adsorbent obtained in step (2), a good solvent for the porous molded body-forming polymer, a porous molded body-forming polymer and, depending on the case, a water-soluble polymer (hydrophilic polymer) are mixed to prepare a slurry. The good solvent for the porous molded body-forming polymer used in step (2) and step (3) is not particularly restricted so long as it stably dissolves the porous molded body-forming polymer at greater than 1 mass% under the production conditions for the porous molded body, and any conventionally known one may be used. Examples of good solvents include N-methyl-2-pyrrolidone (NMP), N,N-dimethylacetamide (DMAC) and N,N-dimethylformamide (DMF). The good solvent used may be a single one alone, or two or more may be used in admixture.

The amount of porous molded body-forming polymer added in step (3) may be such that the proportion of porous molded body-forming polymer/(porous molded body-forming polymer + water-soluble polymer + good solvent for porous molded body-forming polymer) is preferably 3 mass% to 40 mass% and more preferably 4 mass% to 30 mass%. If the porous molded body-forming polymer content is 3 mass% or greater a porous molded body with high strength can be obtained, and if it is 40 mass% or lower, a porous molded body with high porosity can be obtained.

While addition of a water-soluble polymer is not absolutely necessary in step (3), addition can yield a homogeneous porous molded body comprising a filamentous structure that forms a three-dimensional connected network structure on the outer surface and interior of the porous molded body, or in other words, a porous molded body can be obtained with easier pore size control and reliable ion adsorption even with high-speed liquid flow treatment.

The water-soluble polymer used in step (3) is not particularly restricted so long as it is compatible with the good solvent for the porous molded body-forming polymer, and with the porous molded body-forming polymer. A natural polymer, semisynthetic polymer or synthetic polymer may be used as the water-soluble polymer. Examples of natural polymers include guar gum, locust bean gum, carrageenan, gum arabic, tragacanth, pectin, starch, dextrin, gelatin, casein and collagen. Examples of semisynthetic polymers include methyl cellulose, ethyl cellulose, hydroxyethyl cellulose, ethylhydroxyethyl cellulose, carboxymethyl starch and methyl starch. Examples of synthetic polymers include polyvinyl alcohol, polyvinylpyrrolidone (PVP), polyvinyl methyl ether, carboxyvinyl polymer, sodium polyacrylate, and polyethylene glycols such as tetraethylene glycol and triethylene glycol. A synthetic polymer is preferred from the viewpoint of increasing the loading capacity of the inorganic ion adsorbent, while polyvinylpyrrolidone (PVP) or a polyethylene glycol is preferred from the viewpoint of increasing the porosity.

The weight-average molecular weight of the polyvinylpyrrolidone (PVP) or polyethylene glycol is preferably 400 to 35,000,000, more preferably 1,000 to 1,000,000 and even more preferably 2,000 to 100,000. If the weight-average molecular weight is 400 or greater, a porous molded body with high surface openness will be obtained, and if it is 35,000,000 or lower, the viscosity of the slurry during molding will be low, tending to facilitate the molding. The weight-average molecular weight of the water-soluble polymer can be measured by dissolving the water-soluble polymer in a predetermined solvent and analyzing it by gel permeation chromatography (GPC).

The amount of water-soluble polymer added may be such that the proportion of water-soluble polymer/(water-soluble polymer + porous molded body-forming polymer + good solvent for porous molded body-forming polymer) is preferably 0.1 mass% to 40 mass%, more preferably 0.1 mass% to 30 mass% and even more preferably 0.1 mass% to 10 mass%.

If the amount of water-soluble polymer added is 0.1 mass% or greater, it will be possible to uniformly obtain a porous molded body that includes a filamentous structure forming a network structure that is three-dimensionally connected on the outer surface and interior of the porous molded body. If the amount of water-soluble polymer added is 40 mass% or lower, the open area ratio on the outer surface will be satisfactory and the abundance of the inorganic ion adsorbent on the outer surface of the porous molded body will be high, to obtain a porous molded body that can reliably adsorb ions even with high-speed liquid flow treatment.

Step (4): Molding step

In step (4), the slurry obtained in step (3) (molding slurry) is molded. The molding slurry is a mixed slurry comprising the porous molded body-forming polymer, the good solvent for the porous molded body-forming polymer, the inorganic ion adsorbent and if necessary a water-soluble polymer. The form of the porous molded body of the embodiment may be any desired form such as particulate, filamentous, sheet-like, hollow fiber-like, cylindrical or hollow cylindrical, depending on the method of molding the molding slurry.

There are no particular restrictions on the method of molding a particulate form, such as spherical particles, and for example, it may be a rotation nozzle method in which the molding slurry housed in a vessel is ejected from nozzles provided on the side wall of the rotating vessel to form droplets. The rotating nozzle method allows molding into a particulate form with a uniform particle size distribution. More specifically, the method may be atomization of the molding slurry from single-fluid or double-fluid nozzles for coagulation in a coagulating bath.

The nozzle diameters are preferably 0.1 mm to 10 mm and more preferably 0.1 mm to 5 mm. The droplets will be more easily ejected if the nozzle diameters are at least 0.1 mm, and the particle size distribution can be made uniform if it is 10 mm or smaller.

The centrifugal force is represented as the centrifugal acceleration, and it is preferably 5 G to 1500 G, more preferably 10 G to 1000 G and even more preferably 10 G to 800 G. If the centrifugal acceleration is 5 G or greater the formation and ejection of the droplets will be facilitated, and if it is 1500 G or lower the molding slurry will be discharged without becoming filamentous, and widening of the particle size distribution can be inhibited. A narrow particle size distribution will result in uniform water flow channels when the porous molded body is packed into the column, providing an advantage whereby even when ultra high-speed water flow treatment is used there is no leakage of ions (the target of adsorption) from the start of water flow.

A method of molding into a filamentous or sheet form may be a method of extruding the molding slurry from a spinneret or die having that shape, and coagulating it in a poor solvent. A method of molding into a hollow fiber porous molded body may be molding in the same manner as a method of molding the porous molded body into a filamentous or sheet form, but using a spinneret with an annular orifice.

A method of molding the porous molded body into a cylindrical or hollow cylindrical form, when extruding the molding slurry from a spinneret, may be cutting while coagulating in a poor solvent, or coagulation into a filamentous form followed by cutting.

Step (5): Coagulation step

In step (5), the molded article with promoted coagulation obtained in step (4) is further coagulated in a poor solvent to obtain a porous molded body. The poor solvent for step (5) may be a solvent with a solubility of 1 mass% or lower for the porous molded body-forming polymer under the conditions in step (5), and examples include water, alcohols such as methanol and ethanol, ethers, and aliphatic hydrocarbons such as *n*-hexane and *n*-heptane. Water is most preferred as the poor solvent.

In step (5), the good solvent is carried over from the preceding steps, causing variation in the concentration of the good solvent at the start and end points of the coagulation step. The poor solvent may therefore have the good solvent added beforehand, and preferably the coagulation step is carried out while managing the concentration by separate addition of water or the like so as to maintain the initial concentration. By adjusting the concentration of the good solvent it is possible to control the structure (the outer surface open area ratio and particle shapes) of the porous molded body.

When the poor solvent is water or a mixture of water with the good solvent for the porous molded body-forming polymer, the content of the good solvent for the porous molded body-forming polymer with respect to the water in the coagulation step is preferably 0 to 80 mass% and more preferably 0 to 60 mass%. If the content of the good solvent for the porous molded body-forming polymer is 80 mass% or lower, a favorable effect for a satisfactory porous molded body

shape will be obtained.

The temperature of the poor solvent is preferably 40 to 100°C, more preferably 50 to 100°C and even more preferably 60 to 100°C, from the viewpoint of controlling the temperature and humidity of the spaces in the rotating vessel that causes ejection of the droplets by centrifugal force, as described below.

[Production apparatus for porous molded body]

When the porous molded body of the embodiment is in particulate form, the production apparatus comprises a rotating vessel that ejects droplets by centrifugal force and a coagulation tank that stores a coagulating solution, also optionally being provided with a cover that covers the space between the rotating vessel and the coagulation tank and comprising control means that controls the temperature and humidity in the space.

The rotating vessel that ejects droplets by centrifugal force is not restricted to one with a specific construction so long as it has the function of ejecting the molding slurry as spherical droplets by centrifugal force, and examples include known types of rotating discs or rotating nozzles. With a rotating disc, the molding slurry is supplied to the center of the rotating disc and the molding slurry is developed into a film of uniform thickness along the surface of the rotating disc, and then divided into droplets by centrifugal force from the peripheral edges of the disc to eject the microdroplets. A rotating nozzle either has a plurality of through-holes formed in the perimeter wall of a rotating vessel having a hollow disc shape, or it has nozzles attached through the perimeter wall, with the molding slurry being supplied into the rotating vessel while rotating the rotating vessel, and the molding slurry being discharged by centrifugal force from the through-holes or nozzles to form droplets.

The coagulation tank that stores the coagulating solution is not limited to any particular structure so long as it has a function allowing it to store the coagulating solution, and for example, it may be a coagulation tank with an open top side, as is commonly known, or a coagulation tank having a construction in which the coagulating solution is allowed to flow down naturally by gravity along the inner walls of the cylinder situated surrounding the rotating vessel. A coagulation tank with an open top side is an apparatus that allows droplets ejected in the horizontal direction from the rotating vessel to fall down naturally, and traps droplets on the liquid surface of the coagulating solution stored in the open-top coagulation tank. A coagulation tank with a construction in which the coagulating solution is allowed to flow down naturally by gravity along the inner walls of the cylinder surrounding the rotating vessel is an apparatus that discharges the coagulating solution at a roughly equivalent flow rate in the circumferential direction along the inner walls of the cylinder, and traps droplets in the coagulating solution flowing downward along the inner walls, causing them to coagulate.

The control means for the temperature and humidity in the space is provided with a cover that covers the space between the rotating vessel and coagulation tank, and it controls the temperature and humidity in the space. The cover covering the space is not restricted to any particular construction so long as it has the function of isolating the space from the external environment and facilitating practical control of the temperature and humidity in the space, and it may be box-shaped, tubular or umbrella-shaped, for example.

The material of the cover may be stainless steel metal or plastic, for example. For isolation from the external environment, it may also be covered by a known type of insulation. The cover may also be partially provided with openings for temperature and humidity adjustment.

The means for controlling the temperature and humidity in the space is not limited to any particular means so long as it has the function of controlling the temperature and humidity in the space, and for example, it may be a heating machine such as an electric heater or steam heater, or a humidifier such as an ultrasonic humidifier or heating humidifier. A preferred means in terms of construction is one that heats the coagulating solution stored in the coagulation tank and utilizes steam generated from the coagulating solution to control the temperature and humidity in the space.

A method of forming a coating layer of a biocompatible polymer on the surface of a porous molded body will now be described. For this embodiment, a coating solution containing a PMEA- or a PVP-based polymer, for example, may be applied onto the surface of the porous molded body to form a coating film. A PMEA coating solution, for example, can penetrate the pores formed in the porous molded body, allowing the PMEA to be added to the entire porous surface of the porous molded body without significantly altering the pore sizes on the surface of the porous molded article.

The solvent of the PMEA coating solution is not particularly restricted so long as it is a solvent that can dissolve or disperse the PMEA without dissolving the polymers such as the porous molded body-forming polymer of the porous molded body and the water-soluble polymer, but it is preferably water or an aqueous alcohol solution, for process safety and satisfactory handleability in the subsequent drying step. From the viewpoint of boiling point and toxicity it is preferred to use water, an aqueous ethanol solution, an aqueous methanol solution, an aqueous isopropyl alcohol solution, a water/ethanol mixed solvent or a water/methanol mixed solvent. The type and composition of the solvent in the coating solution is selected as appropriate in relation to the polymer forming the porous molded body.

The PMEA concentration of the PMEA coating solution is not restricted, but as an example it may be 0.001 mass% to 1 mass%, and preferably 0.005 mass% to 0.2 mass%, of the coating solution.

The method of applying the coating solution is also not restricted, and an example is a method in which the

porous molded body is packed into a suitable column (vessel) and flushed from the top with a coating solution containing PMEA, and compressed air is then used to remove the excess solution. After subsequently washing with distilled water and substituting out the unnecessary solvent, it may be sterilized for use as a medical tool.

## EXAMPLES

Examples and Comparative Examples will now be described, with the understanding that they are not limitative on the invention. The physical properties of the porous molded body and the performance of the blood purification device were measured as follows. The scope of the invention is not limited to the Examples described below, and various modifications may be implemented within the scope of its gist.

<Evaluation and measuring methods>

[Low-melting-point moisture content]

The "low-melting-point moisture content per gram of dry weight" of the porous molded body was measured by the following procedure.

<Procedure>

1. The weight of an empty pan is measured.
2. The moistened porous molded body is placed in the pan, which is then sealed and weighed.
3. DSC measurement is performed.
4. Following DSC measurement, a small hole is opened in the sealed pan, and vacuum drying is carried out for 8 hours or longer at 80°C.
5. Following the vacuum drying of 4., the pan is weighed.
6. The weight of the empty pan in 1. is subtracted from the weight of the pan after vacuum drying in 5., to calculate the "dry weight of the porous molded body".
7. The weight of the vacuum-dried pan in 5. is subtracted from the weight of the pan in 2., to calculate the total water content of the porous molded body.
8. The heat flow after DSC measurement (ordinate in the graph) is normalized by the total water content.
9. In the absorption (endothermic) peak area in DSC measurement (see Fig. 2), 0.18°C or higher is defined as the heat of fusion of bulk water (total heat of fusion), and lower than 0.18°C is defined as the heat of fusion of low-melting-point water (low-melting-point water heat of fusion).
10. The "low-melting-point moisture content" is calculated by multiplying the total water content by the low-melting-point water percentage (low-melting-point water heat of fusion/total heat of fusion) obtained by DSC.
11. The "low-melting-point moisture content" is divided by the "dry weight of the porous molded body" to calculate the "low-melting-point moisture content per gram of dry weight".

<Devices used>

Apparatus: DSC Q2000 by TA Instruments, or equivalent
Atmosphere: Nitrogen (flow rate: 50 mL/min)
Temperature calibration: Cyclohexane, 6.71°C
Heat quantity calibration: Cyclohexane, 31.9 J/g
Measuring cell: Tzero Hermetic Al Pan (sealed pan)
Reference: Empty Tzero Hermetic Al Pan (empty pan)
Measuring temperature: -40°C to 5°C
Temperature-elevating rate: 0.3°C/min (temperature-lowering rate to -30°C: 3°C/min)
Sample weight measurement: Ultramicro balance by Mettler-Toledo Inc.

[Contact change rate]

The porous molded body in a moist state was loaded into a graduated cylinder. The graduated cylinder was

mechanically tapped a minimum of 20 times, and when no further volume change was seen, an apparent volume of 10 mL of the porous molded body was measured with a scaled graduated cylinder. The 10 mL of the porous molded body was dried for 3 hours at 60°C and the dry weight was measured. A separate 10 mL portion of the porous molded body was also prepared and subjected to suction filtration. The suction filtration was carried out using an aspirator (MDA-015 by Ulvac Co., 5 minutes suction at 0.01 MPa) and filter paper PHWP04700 Mixed Cellulose Ester by Merck Millipore). The total amount of the suction filtered porous molded body was placed in a 200 mL three-necked flask using a funnel. A whole pipette was used to measure out 100 mL of water for injection by Otsuka Pharmaceutical Co., Ltd. and add it to the three-necked flask. A three-one motor, clamp, stirring shaft and stirring blade were mounted on a stand and set on the flask. The stirring blade used was a PTFE square-type, Catalog #1-7733-01 by As One Corp., having a lateral width of 52 mm, a longitudinal width of 14 mm and a thickness of 3.2 mm. The stirring shaft was set at the center of the three-necked flask and the stirring blade was set to extend 3 mm from the water surface. Stirring was then carried out with the three-one motor at 400 rpm for 1 hour. Two sheets of filter paper were prepared that had filtered 100 mL of water for injection by suction filtration, and the two sheets were dried at 60°C for 3 hours and their weights were measured 3 times, recording the average value. An AUW120D fine balance by Shimadu was used for the weight measurement. After stirring the solution at the end of the experiment, the two sheets of filter paper were used for filtration. Care was taken so that the porous molded body did not flow out from the three-necked flask. Next, the flask was rinsed twice while wetting the entire wall with 50 mL of water for injection × 4, and the funnel was rinsed twice. The filter paper was dried at 80°C for 3 hours, the weight was measured 3 times and the average value was calculated. The weight obtained by subtracting the weight of the filter paper from this weight was recorded as the contact change weight. The value derived from the following formula was used as the contact change rate (%).

$$\text{Contact change rate (\%)} = \{\text{Contact change weight}/(\text{apparent 10 mL-volume porous molded body dry weight})\} \times 100$$

The measurement was performed 10 times, and the average value was calculated for 8 measurements, discarding the maximum and minimum.

[Mean particle size of porous molded body and mean particle size of inorganic ion adsorbent]

The mean particle size of the porous molded body and the mean particle size of the inorganic ion adsorbent were measured using a laser diffraction/scattering particle size distribution analyzer (LA-950, trade name of Horiba Co.). The dispersing medium used was water. For measurement of samples using hydrated cerium oxide as the inorganic ion adsorbent, the refractive index used was the value for cerium oxide. Likewise, for measurement of samples using hydrated zirconium oxide as the inorganic ion adsorbent, the refractive index used was the value for zirconium oxide.

[Phosphorus adsorption with bovine plasma]

The apparatus shown in Fig. 1 was used to measure the phosphorus adsorption by a column flow test with low-phosphorus serum using bovine plasma. Bovine plasma prepared to a low phosphorus level (0.7 mg/dL) was used for measurement of the amount of phosphorus adsorbed by the porous molded body (mg-P/mL-resin (porous molded body)) packed into a column (vessel) under conditions equivalent to common dialysis conditions (space velocity SV = 120, 4 hours dialysis).

The phosphate ion concentration was measured by the molybdic acid direct method.

Phosphorus adsorption of 1.5 (mg-P/mL-resin) or greater with a flow speed of SV120 was judged to be high adsorption capacity and satisfactory as a phosphorus adsorbent.

[Amount of microparticles]

A microparticle counter (KL-04 by Rion Co., Ltd.) was used for measurement of each evaluation sample. After discarding the first measured value, measurement was performed an additional 3 times and the average was recorded as the measured value.

[Presence or absence of hemolysis]

The apparatus shown in Fig. 1 was used to measure the phosphorus adsorption by a column flow test with human blood.

An 8 mL portion of the porous molded body weighed out using a graduated cylinder by repeated tapping was packed into a column (inner diameter: 10 mm), and human blood (fresh human blood within 3 hours of blood collection, containing anticoagulant, hematocrit: 40 to 46%) was passed through in a single pass at a rate of 960 mL/hr (SV120 $hr^{-1}$).

The blood running off from the column (treated blood) was sampled every 2 minutes, for a total of 3 times. The run-off blood sampled 3 times was subjected to a hemolysis test by the following method, and judged to have hemolysis if hemolysis was present in any single sample.

(Hemolysis test method)

Human blood before and after filtration was centrifuged for 15 minutes at 3000 rpm (1700 × g), and coloration of the supernatant portion before and after filtration was observed and compared using white paper as the background, and judged on the following scale:

> Hemolysis: (i) Clear dark redness of blood preparation supernatant after filtration compared to blood preparation supernatant before filtration, or (ii) red coloration of blood preparation supernatant after filtration compared to blood preparation supernatant before filtration;
> No hemolysis: (iii) No red coloration found in blood preparation supernatant after filtration compared to blood preparation supernatant before filtration.

<Example 1>

[Production of inorganic ion adsorbent]

After loading 2000 g of cerium sulfate tetrahydrate (Wako Pure Chemical Industries, Ltd.) in 50 L of purified water, a stirring blade was used for dissolution, and then 3 L of 8 M caustic soda (Wako Pure Chemical Industries, Ltd.) was added dropwise at a rate of 20 ml/min to obtain a hydrated cerium oxide precipitate. The obtained precipitate was filtered with a filter press and then washed by flowing through 500 L of purified water, after which 80 L of ethanol (Wako Pure Chemical Industries, Ltd.) was additionally flowed through, replacing the water in the hydrated cerium oxide with ethanol. A 10 ml portion of the filtrate was sampled after filtration was complete, and the moisture content was measured with a Karl Fischer moisture content meter (CA-200, trade name of Mitsubishi Chemical Holdings Corp. Analytech Co., Ltd.), resulting in a moisture content of 5 mass% and an organic liquid replacement rate of 95 mass%. The hydrated cerium oxide containing the organic liquid was air dried to obtain dried hydrated cerium oxide.

[Production of porous molded body]

The obtained dried hydrated cerium oxide was pulverized using a jet mill apparatus (SJ-100, trade name of Nisshin Engineering Inc.) under conditions with a pneumatic pressure of 0.8 MPa and a starting material feed rate of 100 g/hr, to obtain hydrated cerium oxide powder having a mean particle size of 1.2 μm. After adding 220 g of dimethyl sulfoxide (DMSO, product of Kanto Kagaku Co., Ltd.), 120 g of pulverized hydrated cerium oxide powder (MOX), 28 g of poly(methyl methacrylate) (PMMA, trade name: DIANAL BR-77 by Mitsubishi Chemical Corp.) and 32 g of polyvinylpyrrolidone (PVP, K90 by BASF Corp.) as a hydrophilic polymer (water-soluble polymer), the mixture was heated to 60°C in a dissolution tank and a stirring blade was used for stirring to dissolution, to obtain a homogeneous molding slurry solution. The obtained molding slurry was supplied into a cylindrical rotating vessel with 4 mm-diameter nozzles opened in the side wall, and the vessel was rotated to form droplets from the nozzles by centrifugal force (15 G). The droplets were allowed to splash into a coagulation tank with an open top side storing a coagulating solution with an NMP content of 50 mass% with respect to water, that had been heated to 60°C, to coagulate the molding slurry. Alkali cleaning and sorting were also carried out after ethanol replacement, to obtain a spherical porous molded body. The particle size of the porous molded body was 537 μm.

[Washing with supercritical fluid]

The obtained porous molded body was washed for 1 hour using a supercritical fluid comprising carbon dioxide (critical temperature: 304.1K, critical pressure: 7.38 MPa, device by ITEC Co., Ltd.).

[PMEA coating]

A 1 mL portion of the obtained porous molded body was packed into a cylindrical vessel (having a glass filter set at the base, L (length)/D (cylinder diameter) = 1.5). Next, 0.2 g of PMEA (Mn 20,000, Mw/Mn 2.4) was dissolved in

an aqueous solution of 40 g methanol/60 g water (100 g) to prepare a coating solution. The vessel packed with the porous molded body was held vertically and flushed from the top with the coating solution at a flow rate of 100 mL/min, contacting the coating solution with the porous molded body, after which it was washed with purified water. After the purified water washing, the coating solution was sprayed into the vessel with air at 0.1 KMPa, the module was placed in a vacuum dryer and vacuum dried for 15 hours at 35°C, and gamma sterilization was carried out at 25 kGy in an air atmosphere to fabricate a blood purification device.

[Column flow test with low-phosphorus serum using bovine plasma]

Considering the intended use as a phosphorus adsorber after use of a dialyzer in dialysis treatment, it was decided to measure the phosphorus adsorption at a dialyzer outlet during dialysis treatment, with an inorganic phosphorus concentration of 0.2 to 1.0 mg/dL in blood. The phosphorus concentration in the test plasma solution was therefore adjusted. Commercially available bovine serum was centrifuged (3500 rpm, 5 min) and 2000 mL of blood plasma supernatant was prepared. The phosphorus concentration in the blood plasma was 10.8 mg/dL. The porous molded body obtained in Example 1 was added to half of the obtained blood plasma (1000 mL), and stirred for 2 hours at room temperature, after which it was centrifuged (3500 rpm, 5 min) to obtain approximately 950 mL of blood plasma with a phosphorus concentration of 0. After mixing 35 mL of blood plasma with a phosphorus concentration of 10.8 mg/dL and 465 mL of blood plasma with a phosphorus concentration of 0, the mixture was centrifuged (3500 rpm, 5 min) to obtain 495 mL of blood plasma with a phosphorus concentration of 0.8 mg/dL, as supernatant.

Using the porous molded body obtained in Example 1, the blood purification device was incorporated as shown in Fig. 1, and 450 mL of the obtained blood plasma was flowed through at a flow rate of 2 mL/min, sampling 10 mL as the first fraction and 20 mL for each sample thereafter. Based on usual average dialysis conditions of 4 hours of dialysis at a flow rate Qb = 200 mL/min, the total blood flow was 200 mL $\times$ 4 hours = 48,000 mL/min, and assuming the blood cell component to be Ht = 30%, the blood plasma flow was 33,600 mL/min. The amount of liquid flow was 340 mL/min in this case, since the experiment was at a 1/100 scale.

The phosphorus adsorption of the porous molded body at a blood plasma flow volume of 350 mL/min was 1.54 mg-P/mL-resin. The low-melting-point moisture content per gram of dry weight of the obtained porous molded body was 0.62 g. The performance of the obtained blood purification device is shown in Table 1. The blood purification device was safely usable, and had high phosphorus adsorption capacity, satisfactory cytokine adsorption performance, no hemolysis and a microparticle count satisfying the approval standards for artificial kidney devices.

<Example 2>

The procedure was carried out in the same manner as Example 1, according to [Production of porous molded body], but adding 217.6 g of N-methyl-2-pyrrolidone (NMP, product of Mitsubishi Chemical Corp.) as a good polymer resin solvent, 31.6 g of polyvinylpyrrolidone (PVP, K90 by BASF Corp.) as a hydrophilic polymer (water-soluble polymer), 119.2 g of lanthanum oxide (product of Nacalai Tesque, Inc.) instead of MOX, and 31.6 g of polyether sulfone (PES, product of Sumitomo Chemical Co., Ltd.) as a polymer resin, to obtain a spherical porous molded body. The particle size of the porous molded body was 533 $\mu$m. The low-melting-point moisture content per gram of dry weight of the obtained porous molded body was 0.14 g. The phosphorus adsorption was 9.88 mg-P/mL-resin. The performance of the obtained blood purification device is shown in Table 1. The blood purification device was safely usable, and had high phosphorus adsorption capacity, satisfactory cytokine adsorption performance, no hemolysis and a microparticle count satisfying the approval standards for artificial kidney devices.

<Example 3>

The procedure was carried out in the same manner as Example 1, according to [PMEA coating], except that 1.0 g of PMEA was dissolved in an aqueous solution (100 g) of 40 g methanol/60 g water for formation of the coating solution, to obtain a spherical porous molded body. The properties of the obtained blood purification device are shown in Table 1. The low-melting-point moisture content per gram of dry weight of the obtained porous molded body was 1.30 g. The phosphorus adsorption was 1.55 mg-P/mL-resin. The performance of the obtained blood purification device is shown in Table 1. The blood purification device was safely usable, and had high phosphorus adsorption capacity, satisfactory cytokine adsorption performance, no hemolysis and a microparticle count satisfying the approval standards for artificial kidney devices.

<Comparative Example 1>

The procedure was carried out in the same manner as Example 2, though without PMEA coating, to obtain a

spherical porous molded body. The performance of the obtained blood purification device is shown in Table 1. The low-melting-point moisture content per gram of dry weight of the obtained porous molded body was 0.10 g. The low-melting-point moisture content was low and hemolysis was present in the porous molded body.

<Comparative Example 2>

The procedure was carried out in the same manner as Example 1, according to [PMEA coating], except that 1.2 g of PMEA was dissolved in an aqueous solution (100 g) of 40 g methanol/60 g water for formation of the coating solution, to obtain a spherical porous molded body. The performance of the obtained blood purification device is shown in Table 1. The low-melting-point moisture content per gram of dry weight of the obtained porous molded body was 1.40 g. The phosphorus adsorption was 1.45 mg-P/mL-resin. The low-melting-point moisture content was too high, resulting in low phosphorus adsorption.

<Comparative Example 3>

A blood purification device was fabricated in the same manner as Example 1, except that washing with a supercritical fluid was not carried out. The properties of the obtained blood purification device are shown in Table 1. The results showed a large number of microparticles.

<Example 4>

[Synthesis of hydrophilic polymer (biocompatible polymer)]

A copolymer of 2-methoxyethyl methacrylate (MEMA) and N-methacryloyloxyethyl-N,N-dimethylammonium-α-N-methylcarboxybetaine (CMB) was synthesized by common solution polymerization. The polymerization conditions were a concentration of 1 mol/L for each monomer, in an ethanol solution in the presence of 0.0025 mol/L of azoisobutyronitrile (AIBN) as an initiator, and polymerization reaction was conducted for 8 hours at a reaction temperature of 60°C, to obtain a polymer solution. The obtained polymer solution was dropped into diethyl ether and the precipitated polymer was recovered. The recovered polymer was purified by a reprecipitation procedure using diethyl ether. The obtained polymer was then dried for 24 hours under reduced pressure conditions to obtain a hydrophilic polymer (biocompatible polymer).

The molar ratio of HEMA monomer units and CMB monomer units in the hydrophilic polymer (biocompatible polymer) was measured in the following manner. The obtained hydrophilic polymer (biocompatible polymer) was dissolved in dimethyl sulfoxide, and then calculation was performed by the following formula from the peak at 4.32 ppm (from H atoms unique to CMB) and the area ratio at 0.65 to 2.15 ppm (total H atoms), in a chart calculated after carrying out $^1$H-NMR measurement.

$$\text{CMB monomer molar ratio} = (\text{“Area ratio in 4.32 ppm range”}/2)/(\text{“area ratio in 0.65 to 2.15 ppm range”}/5) \times 100$$

$$\text{HEMA monomer molar ratio} = 100 - \text{CMB monomer molar ratio}$$

The molar ratio of HEMA monomer units and CMB monomer units in the hydrophilic polymer (biocompatible polymer) was calculated to be 65:35.

[Preparation of coating solution]

After adding the hydrophilic polymer (biocompatible polymer) to 70 W/W% ethyl alcohol, the mixture was stirred for 12 hours to prepare a coating solution with a coating polymer concentration of 0.1 wt%.

The procedure was carried out in the same manner as Example 2, except that this coating solution was used to produce a blood purification device by the method described under [PMEA coating]. The properties of the obtained blood purification device are shown in Table 1. The low-melting-point moisture content per gram of dry weight of the obtained porous molded body was 1.20 g. The phosphorus adsorption was 9.86 mg-P/mL-resin. The performance of the obtained blood purification device is shown in Table 1. The blood purification device was safely usable, and had high phosphorus adsorption capacity, satisfactory cytokine adsorption performance, no hemolysis, and a microparticle

count satisfying the approval standards for artificial kidney devices.

<Example 5>

The procedure was carried out in the same manner as Example 4, except that polyetherimide (PEI, General Electric Co. Ultem1010) was used as the polymer resin in [Production of porous molded body]. The properties of the obtained blood purification device are shown in Table 1. The low-melting-point moisture content per gram of dry weight of the obtained porous molded body was 1.20 g. The phosphorus adsorption was 9.84 mg-P/mL-resin. The performance of the obtained blood purification device is shown in Table 1. The blood purification device was safely usable, and had high phosphorus adsorption capacity, satisfactory cytokine adsorption performance, no hemolysis, and a microparticle count satisfying the approval standards for artificial kidney devices.

<Example 6>

The procedure was carried out in the same manner as Example 5, except that 240 g of hydrated zirconium oxide (trade name: R Zirconium Hydroxide by Daiichi Kigenso Kagaku Kogyo Co., Ltd.) was used instead of 119.2 g of lanthanum oxide in [Production of porous molded body]. The properties of the obtained blood purification device are shown in Table 1. The low-melting-point moisture content per gram of dry weight of the obtained porous molded body was 1.20 g. The phosphorus adsorption was 1.50 mg-P/mL-resin. The performance of the obtained blood purification device is shown in Table 1. The blood purification device was safely usable, and had high phosphorus adsorption capacity, satisfactory cytokine adsorption performance, no hemolysis, and a microparticle count satisfying the approval standards for artificial kidney devices.

<Example 7>

The procedure was carried out in the same manner as Example 6, except that in [Production of porous molded body], a copolymer with limiting viscosity [$\eta$] = 1.2 (organic polymer resin, PAN), comprising 91.5 wt% acrylonitrile, 8.0 wt% methyl acrylate and 0.5 wt% sodium methacryl sulfonate was used as the polymer resin, and DMSO was used as the good solvent for the polymer resin. The properties of the obtained blood purification device are shown in Table 1. The low-melting-point moisture content per gram of dry weight of the obtained porous molded body was 1.20 g. The phosphorus adsorption was 1.52 mg-P/mL-resin. The performance of the obtained blood purification device is shown in Table 1. The blood purification device was safely usable, and had high phosphorus adsorption capacity, satisfactory cytokine adsorption performance, no hemolysis, and a microparticle count satisfying the approval standards for artificial kidney devices.

<Example 8>

The procedure was carried out in the same manner as Example 7, except that in [Production of porous molded body], neodymium carbonate (trade name: Neodymium Carbonate Octahydrate, product of Fujifilm Wako Chemical Corp.) was used instead of hydrated zirconium oxide. The properties of the obtained blood purification device are shown in Table 1. The low-melting-point moisture content per gram of dry weight of the obtained porous molded body was 1.20 g. The phosphorus adsorption was 9.22 mg-P/mL-resin. The performance of the obtained blood purification device is shown in Table 1. The blood purification device was safely usable, and had high phosphorus adsorption capacity, satisfactory cytokine adsorption performance, no hemolysis, and a microparticle count satisfying the approval standards for artificial kidney devices.

<Example 9>

The procedure was carried out in the same manner as Example 4, except that in [Production of porous molded body], neodymium carbonate (trade name: Neodymium Carbonate Octahydrate, product of Fujifilm Wako Chemical Corp.) was used instead of lanthanum oxide. The properties of the obtained blood purification device are shown in Table 1. The low-melting-point moisture content per gram of dry weight of the obtained porous molded body was 1.20 g. The phosphorus adsorption was 9.25 mg-P/mL-resin. The performance of the obtained blood purification device is shown in Table 1. The blood purification device was safely usable, and had high phosphorus adsorption capacity, satisfactory cytokine adsorption performance, no hemolysis, and a microparticle count satisfying the approval standards for artificial kidney devices.

<Example 10>

The procedure was carried out in the same manner as Example 9, except that in [Production of porous molded body], polysulfone (P-1700 by Amoco Engineering Polymers) was used as the polymer resin. The properties of the obtained blood purification device are shown in Table 1. The low-melting-point moisture content per gram of dry weight of the obtained porous molded body was 1.20 g. The phosphorus adsorption was 9.26 mg-P/mL-resin. The performance of the obtained blood purification device is shown in Table 1. The blood purification device was safely usable, and had high phosphorus adsorption capacity, satisfactory cytokine adsorption performance, no hemolysis, and a microparticle count satisfying the approval standards for artificial kidney devices.

<Example 11>

The procedure was carried out in the same manner as Example 3, except that in [Production of porous molded body], ethylene-vinyl alcohol copolymer (EVOH, trade name: SOARNOL E3803, product of Nippon Synthetic Chemical Industry Co., Ltd.) was used as the polymer resin. The properties of the obtained blood purification device are shown in Table 1. The low-melting-point moisture content per gram of dry weight of the obtained porous molded body was 1.20 g. The phosphorus adsorption was 1.55 mg-P/mL-resin. The performance of the obtained blood purification device is shown in Table 1. The blood purification device was safely usable, and had high phosphorus adsorption capacity, satisfactory cytokine adsorption performance, no hemolysis, and a microparticle count satisfying the approval standards for artificial kidney devices.

<Comparative Example 4>

[Production of porous molded body]

After loading 110 g of N-methyl-2-pyrrolidone (NMP, Mitsubishi Chemical Corp.) and 150 g of hydrated cerium oxide powder with a mean particle size of 30 $\mu$m (Konan Muki Co., Ltd.) into a 1 L-volume stainless steel ball mill pot filled with 1.5 kg of stainless steel balls with diameters of 5 mmcp, pulverizing and mixing treatment was carried out for 150 minutes at a rotational speed of 75 rpm to obtain a yellow slurry. After adding 15 g of polyethersulfone (Sumitomo Chemical Co., Ltd., SUMIKA EXCEL 5003PS (trade name), OH terminal grade, terminal hydroxyl composition: 90 (mol%)) and 2 g of polyethylene glycol (PEG35,000, Merck, Ltd.) as a water-soluble polymer to the obtained slurry, the mixture was heated to 60°C in a dissolution tank and stirred to dissolution using a stirring blade to obtain a homogeneous molding slurry solution.

The obtained molding slurry solution was heated to 60°C and supplied into a cylindrical rotating vessel with 5 mm-diameter nozzles opened in the side wall, and the vessel was rotated to form droplets from the nozzles by centrifugal force (15 G). Next, the space between the rotating vessel and the coagulation tank was covered with a polypropylene cover, and the contents in the space controlled to a temperature of 50°C and a relative humidity of 100% were caused to fly up and land in a coagulation tank with an open top side, which was storing water heated to 80°C as a coagulating solution, to coagulate the molding slurry. Washing and sorting were also carried out to obtain a spherical porous molded body.

[PMEA coating of porous molded body]

A 50 mL portion of the obtained porous molded body was packed into a cylindrical column (having a glass filter set at the base). Next, 0.2 g of PMEA (Mn 20,000, Mw/Mn 2.4) was dissolved in an aqueous solution of 40 g ethanol/60 g water (100 g) to prepare a coating solution. The column packed with the porous molded body was held vertically and flushed from the top with the coating solution at a flow rate of 100 mL/min, contacting the coating solution with the porous molded body, after which it was washed with purified water. After the purified water washing, the coating solution was sprayed into the module with air at 0.1 KMPa, the module was placed in a vacuum dryer and vacuum dried for 15 hours at 35°C, and gamma sterilization was carried out at 25 kGy in an air atmosphere.

As a result of measuring the low-melting-point moisture content and contact change rate of the obtained porous molded body, the low-melting-point moisture content was 0.01 g and the contact change rate was 0.4%. Since the polyethylene glycol used in the molding slurry solution of the porous molded body (PEG35,000, Merck, Ltd.) was water-soluble, it did not remain in the porous molded body. As a result of confirming the amount of PMEA in the porous molded body by ATR-IR, the amount of PMEA in the porous molded body was found to be about 25% compared to Example 1 of the present application.

<Comparative Example 5>

A spherical porous molded body was obtained in the same manner as described in Comparative Example 4, except that pulverizing and mixing treatment were carried out for 200 minutes with 147 g of NMP and 80.5 g of hydrated cerium oxide powder (Konan Muki Co., Ltd.), and 21.3 g of polyethersulfone (Sumitomo Chemical Co., Ltd., SUMIKA EXCEL 5003PS (trade name), OH terminal grade, terminal hydroxyl composition: 90 (mol%)) and 21.3 g of polyvinylpyrrolidone (PVP, Luvitec K30 Powder (trade name) by BASF Japan) as a water-soluble polymer instead of polyethylene glycol were added to the obtained slurry.

As a result of measuring the low-melting-point moisture content and contact change rate of the obtained porous molded body, the low-melting-point moisture content was 0.01 g and the contact change rate was 0.4%. Since the polyvinylpyrrolidone (PVP, Luvitec K30 Powder (trade name) by BASF Japan; mentioned in Example 2) used in the molding slurry solution of the porous molded body was water-soluble, it did not remain in the porous molded body. As a result of confirming the amount of PMEA in the porous molded body by ATR-IR, the amount of PMEA in the porous molded body was found to be about 25% compared to Example 1 of the present application.

<Effect of solvent in PMEA coating solution>

In Comparative Examples 4 and 5, an aqueous solution of 40 g ethanol/60 g water was used as the PMEA coating solution. For Examples 1 to 3 and 11 of the present application, however, an aqueous solution of 40 g methanol/60 g water was used. Fig. 3 is a diagram showing PMEA solubility with PMEA coating solution solvents. Fig. 4 shows an example of ATR/FT-IR analysis of a porous molded body that includes polyethersulfone (PES) and MOX, after PMEA coating. In Fig. 4, C1 represents the peak due to the C=C bond of PES, and C2 represents the peak due to the C=O bond of PMEA. Fig. 5 shows differences in PMEA coating amounts with PMEA coating solution solvents. It is seen that even with approximately the same PMEA concentration, large differences in coating amounts (up to 4 times greater) exist depending on the type of solvent used. The C2/C1 ratio for the coating amount and ATR in UV measurement was found to be the same. Therefore, it is preferred to use a methanol/water mixed solvent as the solvent of the PMEA coating solution, with a methanol:water ratio of preferably 80:20 to 40:60, more preferably 70:30 to 45:55 and even more preferably 60:40 to 45:55.

[Table 1]

Table 1-1

| | Example 1 | Example 2 | Example 3 | Comparative Example 1 | Comparative Example 2 | Comparative Example 3 | Comparative Example 4 |
|---|---|---|---|---|---|---|---|
| Porous molded body-forming polymer/weight (wt%) | PMMA/ 7.0 | PES/7.9 | PMMA/ 7.0 | PES/7.9 | PMMA/7.0 | PMMA/7.0 | PES/7.9 |
| Water-soluble polymer/weight (wt%) | PVP/8.0 | PVP/7.9 | PVP/8.0 | PVP/7.9 | PVP/8.0 | PVP/8.0 | PVP/7.9 |
| Inorganic ion adsorbent/weight (wt%) | Ce/30.0 | La/29.8 | Ce/30.0 | La/29.8 | Ce/30.0 | Ce/30.0 | La/29.8 |
| Solvent/weight (wt%) | DMSO/ 55.0 | NMP/ 54.4 | DMSO/ 55.0 | NMP/54.4 | DMSO/55.0 | DMSO/55.0 | NMP/54.4 |
| Polymer/concentration in coating solution (wt%) | PMEA/ 0.2 | PMEA/ 0.2 | PMEA/1 | - | PMEA/1.2 | PMEA/0.2 | MEMA, CMB/0.1 |
| Particle size of inorganic ion adsorbent (μm) | 1.2 | 1.2 | 1.2 | 1.2 | 1.2 | 1.2 | 1.2 |
| Particle size of porous molded body (μm) | 537 | 533 | 537 | 533 | 536 | 537 | 533 |
| Low-melting-point moisture content (g) | 0.62 | 0.14 | 1.30 | 0.10 | 1.40 | 0.62 | 1.20 |
| Contact change rate (%) | 0.2 | 0.2 | 0.0 | 0.2 | 0.0 | 0.3 | 0.2 |
| Blood phosphorus adsorption (mg/ml-resin) | 1.54 | 9.88 | 1.55 | 9.88 | 1.45 | 1.55 | 9.86 |
| Estimated volume of pores with pore diameters of 5 nm to 100 nm ($cm^3/g$) | 0.5 | 0.55 | 0.5 | 0.55 | 0.5 | 0.5 | 0.5 |
| Estimated volume of pores with pore diameters of 100 nm to 200 nm ($cm^3/g$) | 0.020 | 0.021 | 0.020 | 0.021 | 0.020 | 0.020 | 0.020 |
| Albumin adsorption (mg/mL) | 21 | 20 | 21 | 20 | 21 | 21 | 53 |
| IL-1b adsorption rate (%) | 88 | 96 | 87 | 80 | 88 | 88 | 100 |
| IL-6 adsorption rate (%) | 67 | 77 | 65 | 58 | 66 | 67 | 94 |
| IL-8 adsorption rate (%) | 83 | 96 | 88 | 77 | 85 | 85 | 100 |
| IL-10 adsorption rate (%) | 64 | 71 | 66 | 60 | 65 | 67 | 96 |
| TNF-α adsorption rate (%) | 30 | 38 | 31 | 30 | 31 | 30 | 87 |
| HMGB1 adsorption rate (%) | 94 | 95 | 96 | 90 | 97 | 95 | 100 |
| Presence or absence of hemolysis | No | No | No | Yes | No | No | No |

(continued)

| | | Example 1 | Example 2 | Example 3 | Comparative Example 1 | Comparative Example 2 | Comparative Example 3 | Comparative Example 4 |
|---|---|---|---|---|---|---|---|---|
| Number of microparticles after elapse of 1 week | ≥10 μm [number] | 6 | 8 | 5 | 8 | 3 | 12 | 7 |
| | ≤25 μm [number] | 1 | 2 | 1 | 2 | 0 | 2 | 2 |
| Number of microparticles after elapse of 3 months | ≥10 μm [number] | 8 | 9 | 8 | 10 | 7 | 29 | 8 |
| | ≤25 μm [number] | 1 | 3 | 1 | 3 | 0 | 6 | 3 |
| Number of microparticles after elapse of 6 months | ≥10 μm [number] | 9 | 10 | 8 | 10 | 8 | 64 | 11 |
| | ≤25 μm [number] | 1 | 3 | 1 | 3 | 1 | 11 | 3 |

[Table 2]

Table 1-2

| | Example 5 | Example 6 | Example 7 | Example 8 | Example 9 | Example 10 | Example 11 |
|---|---|---|---|---|---|---|---|
| Porous molded body-forming polymer/weight (wt%) | PEI/7.9 | PEI/6.1 | PAN/6.1 | PAN/7.9 | PES/7.9 | PSF/7.9 | EVOH/ 7.9 |
| Water-soluble polymer/weight (wt%) | PVP/7.9 | PVP/6.1 | PVP/6.1 | PVP/7.9 | PVP/7.9 | PVP/7.9 | PVP/7.9 |
| Inorganic ion adsorbent/weight (wt%) | La/29.8 | Zr/46.1 | Zr/46.1 | Nd/29.9 | Nd/29.8 | Nd/29.8 | Ce/29.8 |
| Solvent/weight (wt%) | NMP/54.4 | NMP/41.8 | DMSO/41.8 | DMSO/54.4 | NMP/54.4 | NMP/54.4 | DMSO/ 54.4 |
| Polymer/concentration in coating solution (wt%) | MEMA, CMB/ 0.1 | MEMA, CMB/ 0.1 | MEMA, CMB/ 0.1 | MEMA, CMB/ 0.1 | MEMA, CMB/ 0.1 | MEMA, CMB/ 0.1 | PMEA/1 |
| Particle size of inorganic ion adsorbent (μm) | 1.2 | 1.2 | 1.2 | 1.2 | 1.2 | 1.2 | 1.2 |
| Particle size of porous molded body (μm) | 536 | 536 | 532 | 533 | 535 | 536 | 530 |
| Low-melting-point moisture content (g) | 1.20 | 1.20 | 1.22 | 1.22 | 1.20 | 1.20 | 1.23 |
| Contact change rate (%) | 0.2 | 0.2 | 0.2 | 0.2 | 0.2 | 0.2 | 0.0 |
| Blood phosphorus adsorption (mg/ml-resin) | 9.84 | 1.50 | 1.52 | 9.22 | 9.25 | 9.26 | 1.55 |
| Estimated volume of pores with pore diameters of 5 nm to 100 nm ($cm^3/g$) | 0.5 | 0.89 | 0.89 | 0.45 | 0.5 | 0.5 | 0.4 |
| Estimated volume of pores with pore diameters of 100 nm to 200 nm ($cm^3/g$) | 0.020 | 0.021 | 0.015 | 0.015 | 0.020 | 0.020 | 0.010 |
| Albumin adsorption (mg/mL) | 53 | 88 | 84 | 37 | 55 | 57 | 25 |
| IL-lb adsorption rate (%) | 100 | 100 | 100 | 100 | 100 | 100 | 88 |
| IL-6 adsorption rate (%) | 95 | 87 | 86 | 95 | 95 | 96 | 67 |
| IL-8 adsorption rate (%) | 100 | 90 | 91 | 100 | 100 | 100 | 88 |
| IL-10 adsorption rate (%) | 95 | 88 | 87 | 96 | 97 | 98 | 67 |
| TNF-α adsorption rate (%) | 88 | 70 | 69 | 86 | 86 | 88 | 30 |
| HMGB1 adsorption rate (%) | 100 | 100 | 100 | 100 | 100 | 100 | 95 |
| Presence or absence of hemolysis | No | No | No | No | No | No | No |

(continued)

| | | Example 5 | Example 6 | Example 7 | Example 8 | Example 9 | Example 10 | Example 11 |
|---|---|---|---|---|---|---|---|---|
| Number of microparticles after elapse of 1 week | ≥10 μm [number] | 7 | 9 | 9 | 7 | 7 | 8 | 5 |
| | ≤25 μm [number] | 1 | 1 | 1 | 1 | 2 | 2 | 1 |
| Number of microparticles after elapse of 3 months | ≥10 μm [number] | 8 | 14 | 13 | 9 | 9 | 10 | 7 |
| | ≤25 μm [number] | 2 | 2 | 2 | 2 | 2 | 2 | 2 |
| Number of microparticles after elapse of 6 months | ≥10 μm [number] | 10 | 17 | 17 | 11 | 11 | 11 | 9 |
| | ≤25 μm [number] | 2 | 3 | 3 | 2 | 3 | 3 | 2 |

INDUSTRIAL APPLICABILITY

Since the blood purification device of the invention has high phosphorus adsorption capacity, no hemolysis and safe usability, it can be suitably used in therapy for periodic removal of phosphorus that has accumulated in the body.

REFERENCE SIGNS LIST

1 Thermostatic bath
2 Laboratory bench
3 Pump
4 Column containing porous absorber (phosphorus absorbent)
5 Pressure gauge
6 Sampling

## Claims

1. A blood purification device comprising a porous molded body having a low-melting-point moisture content of 0.12 g to 1.35 g per gram of dry weight, wherein the number of microparticles of 10 μm or greater is 25 or less, and the number of microparticles of 25 μm or greater is 3 or less, in 1 mL of physiological saline for injection at 3 months and 6 months after sealing the physiological saline for injection in the blood purification device.

2. The blood purification device according to claim 1, wherein the contact change rate of the porous molded body is from 0 to 0.2.

3. The blood purification device according to claim 1, wherein the porous molded body is composed of a porous molded body-forming polymer, a hydrophilic polymer and an inorganic ion adsorbent.

4. The blood purification device according to claim 3, wherein the hydrophilic polymer is a biocompatible polymer.

5. The blood purification device according to claim 4, wherein the biocompatible polymer is a polyvinylpyrrolidone (PVP)-based polymer.

6. The blood purification device according to any one of claims 1 to 5, wherein the porous molded body is coated with a biocompatible polymer.

7. The blood purification device according to any one of claims 3 to 6, wherein the inorganic ion adsorbent contains at least one metal oxide represented by the following formula (1):

$$MN_xO_n \cdot mH_2O \qquad (1)$$

where x is 0 to 3, n is 1 to 4, m is 0 to 6, and M and N are metal elements selected from the group consisting of Ti, Zr, Sn, Sc, Y, La, Ce, Pr, Nd, Sm, Eu, Gd, Tb, Dy, Ho, Er, Tm, Yb, Lu, Al, Si, Cr, Co, Ga, Fe, Mn, Ni, V, Ge, Nb and Ta, and are different from each other.

8. The blood purification device according to claim 7, wherein the metal oxide is selected from among the following groups (a) to (c):

(a) hydrated titanium oxide, hydrated zirconium oxide, hydrated tin oxide, hydrated cerium oxide, hydrated lanthanum oxide and hydrated yttrium oxide;
(b) complex metal oxides comprising at least one metal element selected from the group consisting of titanium, zirconium, tin, cerium, lanthanum and yttrium and at least one metal element selected from the group consisting of aluminum, silicon and iron; and
(c) activated alumina.

9. The blood purification device according to claim 1, having a phosphorus adsorption of 1.5 (mg-P/mL-resin) or greater.

**10.** The blood purification device according to claim 1, having an adsorption rate for cytokines IL-1b, IL-6, IL-8 and IL-10 of 50% or greater.

**11.** The blood purification device according to claim 1, having an adsorption rate for cytokine TNF-$\alpha$ of 30% or greater.

**12.** The blood purification device according to claim 1, having an adsorption rate for alarmin HMGB-1 of 50% or greater.

FIG. 1

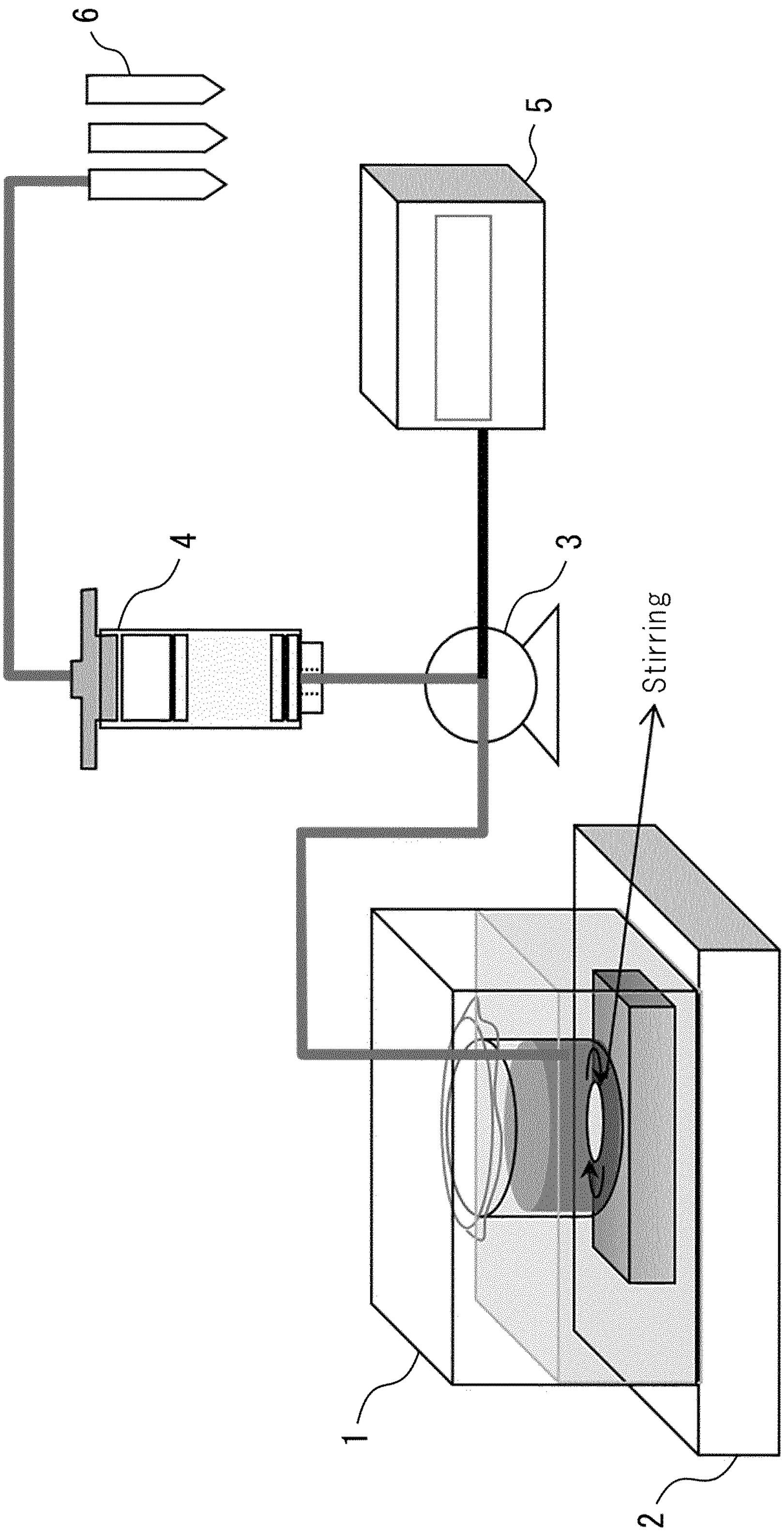

FIG. 2

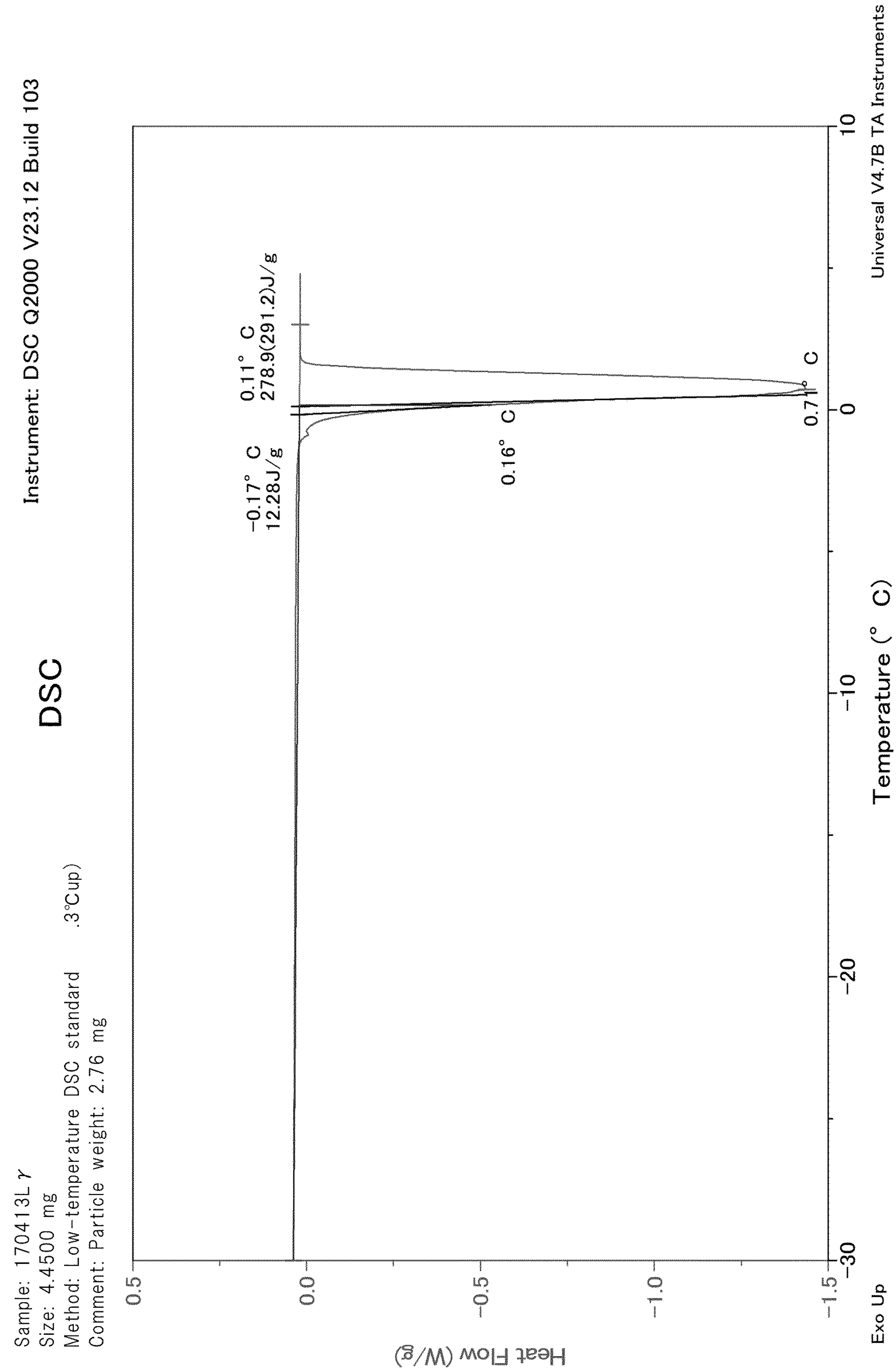
DSC
Sample: 170413L γ
Size: 4.4500 mg
Method: Low-temperature DSC standard .3°Cup)
Comment: Particle weight: 2.76 mg
Instrument: DSC Q2000 V23.12 Build 103
0.11° C
278.9(291.2)J/g
-0.17° C
12.28J/g
0.16° C
0.71° C
Heat Flow (W/g)
Exo Up
Temperature (° C)
Universal V4.7B TA Instruments
0.5
0.0
-0.5
-1.0
-1.5
-30
-20
-10
0
10

FIG. 3

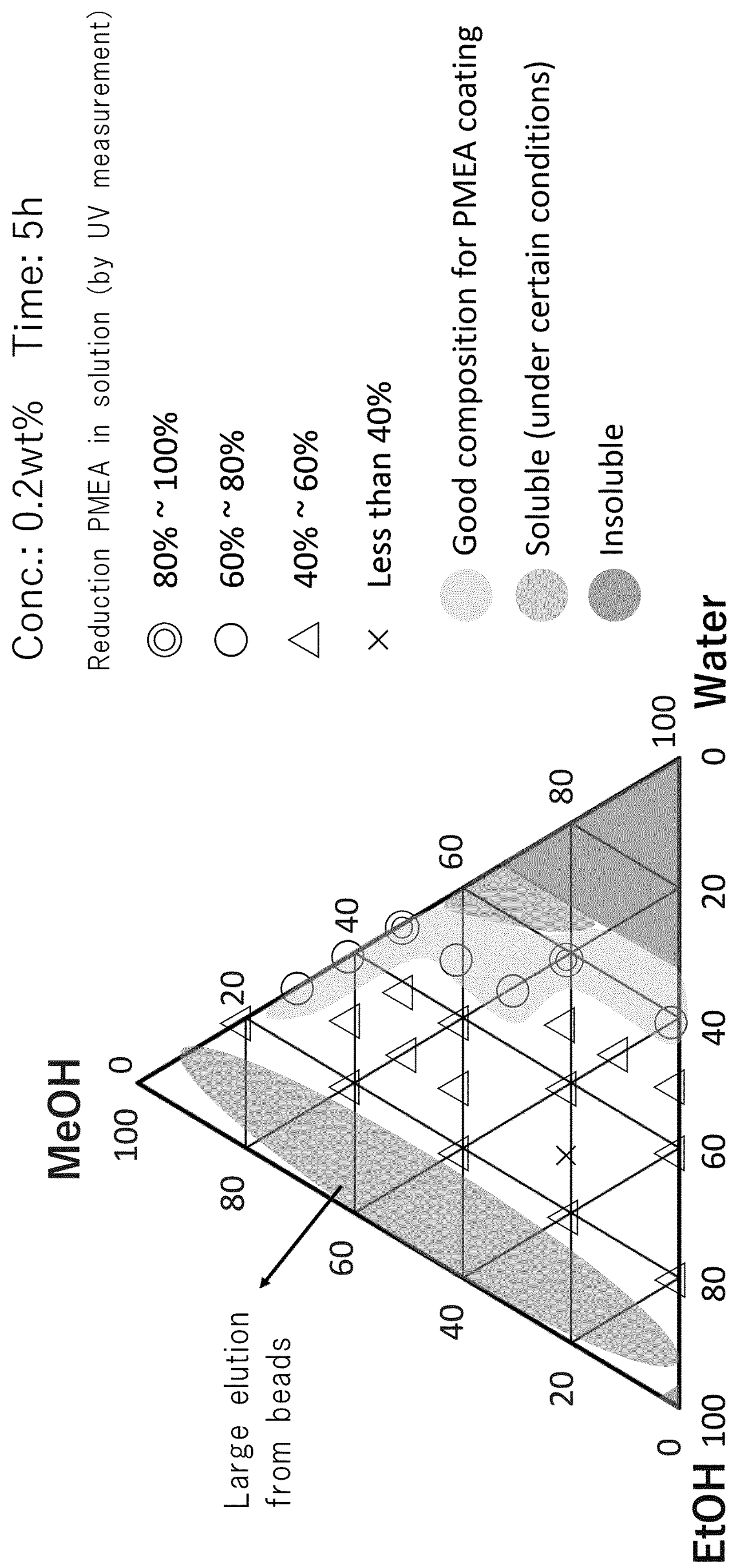

Conc.: 0.2wt%   Time: 5h
Reduction PMEA in solution (by UV measurement)
80% ~ 100%
60% ~ 80%
40% ~ 60%
Less than 40%
Good composition for PMEA coating
Soluble (under certain conditions)
Insoluble
MeOH
Water
EtOH
Large elution from beads
0
20
40
60
80
100

FIG. 4

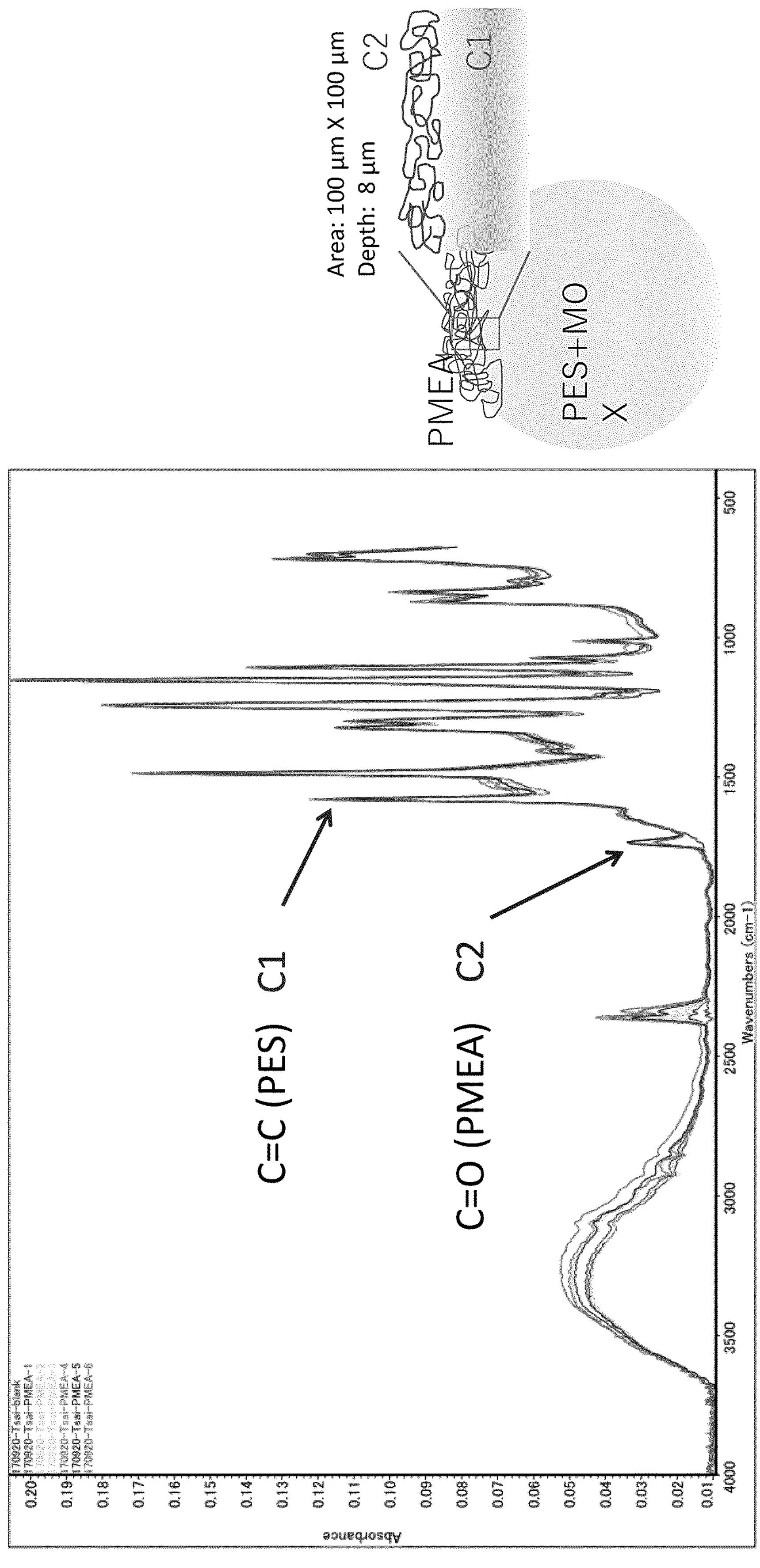
Area: 100 μm X 100 μm
Depth: 8 μm
C2
C1
PMEA
PES+MO
X
C=C (PES) C1
C=O (PMEA) C2
Absorbance
Wavenumbers (cm-1)
170920-Tsai-blank
170920-Tsai-PMEA-1
170920-Tsai-PMEA-2
170920-Tsai-PMEA-3
170920-Tsai-PMEA-4
170920-Tsai-PMEA-5
170920-Tsai-PMEA-6

FIG. 5

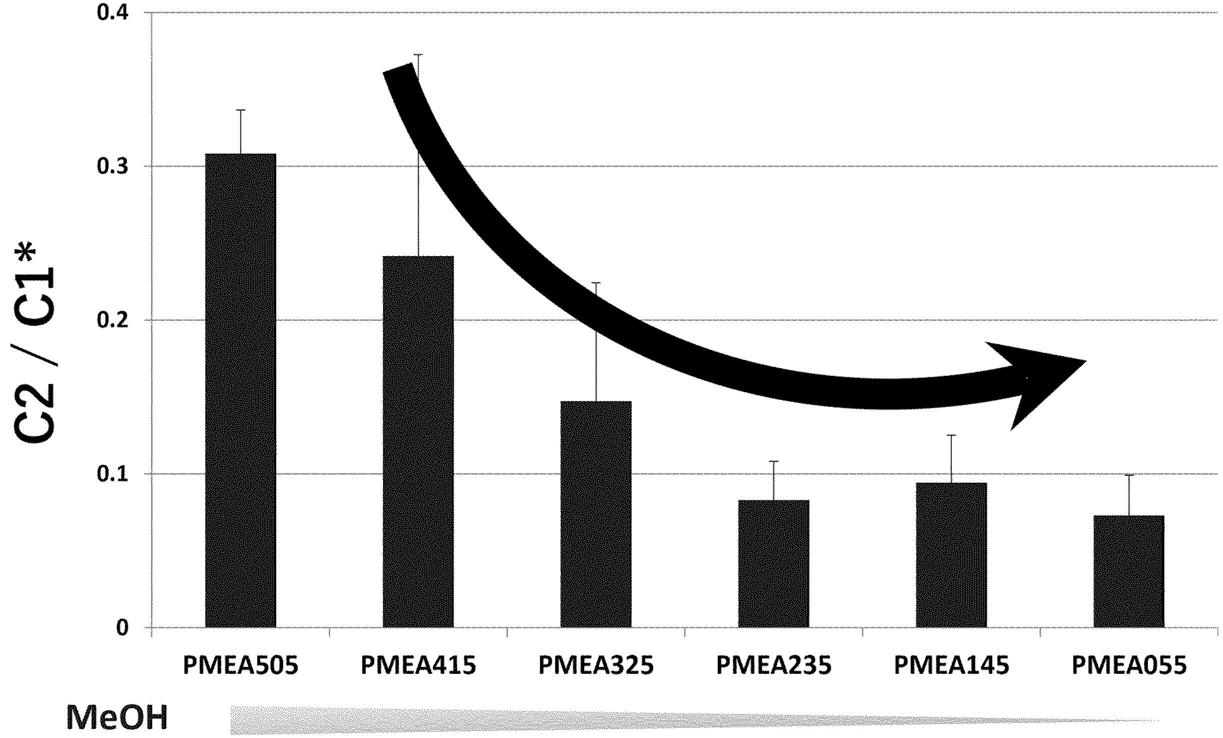


| No. | Solvent |
|---|---|
| 505 | MeOH:EtOH:water = 50 : 0 : 50 |
| 415 | MeOH:EtOH:water= 40 : 10 : 50 |
| 325 | MeOH:EtOH:water = 30 : 20 : 50 |
| 235 | MeOH:EtOH:water = 20 : 30 : 50 |
| 145 | MeOH:EtOH:water = 10 : 40 : 50 |
| 055 | MeOH:EtOH:water = 0 : 50 : 50 |

**INTERNATIONAL SEARCH REPORT**

International application No. PCT/JP2020/014383

A. CLASSIFICATION OF SUBJECT MATTER
Int.Cl. B01J20/06(2006.01)i, B01J20/08(2006.01)i, B01J20/10(2006.01)i, B01J20/28(2006.01)i, B01J20/30(2006.01)i, A61M1/02(2006.01)i, A61M1/36(2006.01)i
FI: A61M1/36165, A61M1/02130, B01J20/08C, B01J20/10C, B01J20/28Z, B01J20/30, B01J20/06B
According to International Patent Classification (IPC) or to both national classification and IPC

B. FIELDS SEARCHED
Minimum documentation searched (classification system followed by classification symbols)
Int.Cl. B01J20/06, B01J20/08, B01J20/10, B01J20/28, B01J20/30, A61M1/02, A61M1/36

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2017-86563 A (ASAHI KASEI MEDICAL CO., LTD.) 25.05.2017 (2017-05-25), paragraphs [0029]-[0035], [0037], [0041], [0042], [0050], [0072], [0073] | 1-5, 7-12<br>6 |
| Y | JP 2010-233999 A (ASAHI KASEI KURARAY MEDICAL CO., LTD.) 21.10.2010 (2010-10-21), paragraph [0058] | 6 |
| Y | JP 2016-77570 A (ASAHI KASEI MEDICAL CO., LTD.) 16.05.2016 (2016-05-16), paragraphs [0047]-[0050] | 6 |
| A | JP 2015-116212 A (ASAHI KASEI MEDICAL CO., LTD.) 25.06.2015 (2015-06-25) | 1-12 |
| A | JP 2015-116214 A (ASAHI KASEI MEDICAL CO., LTD.) 25.06.2015 (2015-06-25) | 1-12 |

☐ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08.06.2020 | 16.06.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/014383

| | | |
|---|---|---|
| JP 2017-86563 A | 25.05.2017 | (Family: none) |
| JP 2010-233999 A | 21.10.2010 | (Family: none) |
| JP 2016-77570 A | 16.05.2016 | (Family: none) |
| JP 2015-116212 A | 25.06.2015 | (Family: none) |
| JP 2015-116214 A | 25.06.2015 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011125758 A
- JP 2002102335 A
- JP 4671419 B


### Non-patent literature cited in the description


- **SHIGEAKI MORITA ; MASARU TANAKA ; YUKIHIRO OZAKI.** Time-Resolved In Situ ATR-IR Observations of the Process of Sorption of Water into a Poly(2-methoxyethyl acrylate) Film. *Langmuir,* 03 March 2007, vol. 23 (7), 3750-3761
- **T. TSURUTA ; J. BIOMATER. et al.** The roles of water molecules in the interfaces between biological systems and polymers. *Sci. Polym. Ed.,* 2010, vol. 21, 1827-1920
- **TANAKA, K.** Artificial organ surface-biocompatibilizing materials. *BIO INDUSTRY,* 2003, vol. 20 (12), 59-70